# EUROPÄISCHE PATENTSCHRIFT

(11) **EP 2 317 911 B1**
(45) Veröffentlichungstag und Bekanntmachung des Hinweises auf die Patenterteilung: **03.10.2018**
(21) Anmeldenummer: 09782043.5
(22) Anmeldetag: 20.08.2009
(51) Int. Cl.: A61B 5/00, A61K 49/00, C08B 37/00, G01N 33/487, G01N 33/58

(54) **TRANSKUTANE ORGANFUNKTIONSMESSUNG**
TRANSCUTANEOUS ORGAN FUNCTION MEASUREMENT
MESURE TRANSCUTANÉE D'UNE FONCTION ORGANIQUE

(30) Priorität: 22.08.2008 EP 08162802
(43) Veröffentlichungstag der Anmeldung: 11.05.2011
(73) Patentinhaber: Medibeacon Inc., St. Louis, MO 63132 (US)
(72) Erfinder: GRETZ, Norbert, 68259 Mannheim (DE); HESSER, Jürgen, 69118 Heidelberg (DE); PILL, Johannes, 69181 Leimen (DE); SCHOCK-KUSCH, Daniel, 68199 Mannheim (DE); SADICK, Maliha, 67454 Hassloch (DE); WALTER, Thomas, 69242 Mühlhausen (DE); EICKEMEYER, Felix, 69118 Heidelberg (DE); HWANG, JAE Hyuug, 68199 Mannheim (DE); WATANABE, Soichi, 68161 Mannheim (DE); SCHILDKNECHT, Christian, 68305 Mannheim (DE); ROSE, Thomas, 67549 Worms (DE); WACH, Wolfgang, 67549 Worms (DE)
(74) Vertreter: Herzog, Fiesser & Partner Patentanwälte PartG mbB
(86) Internationale Anmeldenummer: PCT/EP2009/060785
(87) Internationale Veröffentlichungsnummer: WO 2010/020673

(56) Entgegenhaltungen:
- EP-A1- 1 707 114
- EP-A2- 1 752 085
- WO-A1-01/85799
- WO-A2-2006/032441
- US-A1- 2008 082 004
- PAIS ANDREA ET AL: "High-sensitivity, disposable lab-on-a-chip with thin-film organic electronics for fluorescence detection." LAB ON A CHIP MAY 2008, Bd. 8, Nr. 5, Mai 2008 (2008-05), Seiten 794-800, XP002552745 ISSN: 1473-0197 in der Anmeldung erwähnt
- QI Z ET AL: "Serial determination of glomerular filtration rate in conscious mice using FITC-inulin clearance." AM J PHYSIOL, Bd. 286, Nr. 3 Part 2, März 2004 (2004-03), Seiten F590-F596, XP002570217
- Anonym: "Biochemicals & Reagents for Life Science Research 2004-2005" 2004, Sigma-Aldrich Company Ltd. , Dorset, England , XP002570218Seite 1115, Verbindung Inulin-FITC

## Beschreibung

Die Offenbarung betrifft Sensorpflaster, Sensorsysteme, Kits, sowie deren Verwendungen, sowie ein Verfahren zur Herstellung eines Sensorpflasters, ein Verfahren zur transkutanen Messung einer Organfunktion und eine Verwendung einer Fluoreszenz-markierten Indikatorsubstanz zur Herstellung eines Diagnostikums. Derartige Vorrichtungen und Verfahren können insbesondere zur Messung einer Nierenfunktion eingesetzt werden, insbesondere zur Messung einer glomerulären Filtrationsrate. Auch andere Anwendungen sind jedoch grundsätzlich denkbar.

Im klinischen und präklinischen Bereich kommt der Bestimmung verschiedener Organfunktionen eine große Bedeutung zu, da gemäß diesen Organfunktionen beispielsweise entsprechende Therapien oder Medikationen gesteuert werden können. Die Offenbarung wird im Folgenden im Wesentlichen in Bezug auf die Nierenfunktion beschrieben. Grundsätzlich sind jedoch auch andere Anwendungen denkbar, bei welchen die Funktion eines bestimmten Organs mittels einer Bestimmung eines zeitlichen Verlaufs einer Indikatorsubstanz erfasst werden kann.

In der Nierendiagnostik spielt die quantitative und qualitative Funktionsprüfung der Nieren eine große Rolle. Ein Indikator für die Nierenfunktion ist die so genannte glomeruläre Filtrationsrate (GFR). Hierunter ist indirekt die von den Glomerula der Nieren pro Zeiteinheit produzierte Menge an Primärharn zu verstehen.

Zur Quantifizierung der glomerulären Filtrationsrate sind aus dem Stand der Technik und der medizinischen Praxis mehrere Verfahren bekannt. Eine Klasse von Verfahren, in welche auch die vorliegende Erfindung einzuordnen ist, basiert auf der Verwendung einer oder mehrerer Indikatorsubstanzen. So lassen sich grundsätzlich beliebige exogene oder endogene Substanzen im Blut als Indikatorsubstanzen einsetzen, welche zumindest überwiegend aufgrund der Nierenfunktion aus dem Blut entfernt werden. Dies bedeutet, dass die Entfernung der Indikatorsubstanz aus dem Körper zumindest überwiegend durch die Filtrationswirkung der Glomerula erfolgt, wobei im Wesentlichen weder eine tubuläre Sezernierung noch eine Resorption aus dem Primärharn erfolgt. Die Entfernung der Indikatorsubstanz aus dem Blut wird auch als renale Clearance bezeichnet. Als Clearance wird dabei allgemein diejenige Plasmamenge in Millilitern bezeichnet, die durch die Nieren pro Minute von der Indikatorsubstanz befreit wird.

Zur Bestimmung der renalen Clearance und damit der glomerulären Filtrationsrate sind verschiedene exogene und/oder endogene Indikatorsubstanzen bekannt. Beispiele endogener Indikatorsubstanzen sind Kreatinin oder Cystatin C. Auch verschiedene exogene Indikatorsubstanzen sind aus dem Stand der Technik bekannt. Insbesondere lassen sich als Indikatorsubstanzen Saccharide, z.B. Polyfructosane, einsetzen. Beispiele von geeigneten Indikatorsubstanzen sind in WO2001/85799 oder WO2006/32441 offenbart.

Messtechnisch liegt eine der Herausforderungen insbesondere darin, den Konzentrationsverlauf der Indikatorsubstanz und damit deren Clearance, zu ermitteln. In WO 99/31183 werden zahlreiche verschiedene Verfahren zusammengestellt, mittels derer die Clearance messtechnisch erfasst werden kann. So basieren einige der Verfahren darauf, dass in regelmäßigen oder unregelmäßigen Abständen Blut- und/oder Urinproben genommen werden, und die Konzentration der Markersubstanz analytisch bestimmt wird, beispielsweise über enzymatische Nachweismethoden. Andere Verfahren beruhen auf der Verwendung radioaktiver Indikatorsubstanzen und/oder Röntgenkontrastmitteln. Die Akzeptanz derartiger Indikatorsubstanzen beim Patienten ist jedoch allgemein gering. Allgemein sind Verfahren, die auf einer Bestimmung der renalen Clearance mittels chemischer bzw. biochemischer Analytik beruhen oder auf der Verwendung von radioaktiven Indikatorsubstanzen aufwändig und mit hohen Fehlern belastet. Im klinischen Alltag wird daher in vielen Fällen die Nierenfunktion anhand von Näherungsformeln geschätzt, welche jedoch ebenfalls sehr ungenau sind und Fehlertoleranzen im Bereich von 30 bis 40% aufweisen können.

Ebenfalls aus dem Stand der Technik sind daher Verfahren bekannt, welche auf der Verwendung von Fluoreszenzmarkern beruhen. Dabei werden Indikatorsubstanzen eingesetzt, welche mit Farbstoffen markiert sind, die sich auf optischem Wege nachweisen lassen. Beispielsweise können dies Fluoreszenzmarker sein, welche den Indikatorsubstanzen beigemischt oder mit den Indikatorsubstanzen verbunden werden, beispielsweise durch kovalente Bindung. Beispiele markierter Indikatorsubstanzen sind in WO2001/85799 oder WO2006/32441 beschrieben. In WO 2006/032441 werden verschiedene farbstoffmarkierte Polyole als Verbindungen bereitgestellt, die als Markersubstanzen in der der Nierendiagnostik eingesetzt werden können. Qi et al. (2004), Am J Physiol 286(3) Part 2): F590 offenbaren die Verwendung eines Gemischs aus Inulinen zur Messung der glomerulären Filtrationsrate.

Bei letzteren genannten Verfahren wird somit ein optisches Signal als Maßstab für die Konzentration der Indikatorsubstanz verwendet. Dabei kann beispielsweise aus einer bekannten Relation zwischen dem optischen Signal und der Konzentration auf die jeweilige Konzentration der Indikatorsubstanz geschlossen werden. Diese bekannte Relation kann beispielsweise empirischer, semiempirischer oder analytischer Art sein, beispielsweise eine mittels Kalibrationsmessungen bestimmte Relation. So wird beispielsweise in DE 100 23 051 A1 als Indikatorsubstanz Sinistrin verwendet, welches mit Fluoresceinisothiocyanat (FITC) markiert ist. Dabei wird unter anderem eine nicht-invasive, transkutane Messung des FITC-Fluoreszenzsignals mittels eines nicht-invasiven Messkopfs beschrieben. Dieser Messkopf ist als faseroptischer Messkopf ausgestaltet, bei welchem eine externe Lichtquelle über eine Glasfaser die Haut beleuchtet und die darin enthaltenen FITC-Sinistrin-Moleküle anregt. Das vom FITC ausgesandte Fluoreszenzlicht wird wiederum mittels Glasfasern aufgenommen und einem externen Detektor zugeleitet. EP 1 752 085 A2 schlägt ein Messpflaster zur Verwendung mit einem fluoreszierenden, Licht-emittierenden Bead vor, der unter die die Haut des Anwenders implantiert wird. Eine ähnliche Vorrichtung wird in EP 1 707 114 A1 vorgeschlagen. In US 2008/0082004 A1 wird ein Messpflaster offenbart, welches ein Trägerelement mit Haftoberfläche, eine Strahlenquelle und einen Detektor umfasst, zur Blutdruckmessung offenbart.

Die in DE 100 23 051 A1 und WO 01/85799 A beschriebene Messung der Fluoreszenzsignale ist jedoch apparatetechnisch äußerst aufwändig. So müssen aufwändige Spektrographen bereitgestellt werden, um die Messsignale auszuwerten. Zudem ist eine Faseroptik erforderlich, welche aufgrund der damit verbundenen Verluste des Anregungslichts erzwingt, dass sehr intensive Lichtquellen eingesetzt werden, insbesondere Laser. Die Faseroptik, gemeinsam mit den aufwändigen Lichtquellen und Lasern, bewirkt jedoch, dass eine Messung der renalen Clearance nicht ambulant oder mittels tragbarer Geräte durchgeführt werden kann, sondern praktisch ausschließlich in speziell dafür eingerichteten optischen Labors.

Allgemein sind aus anderen Bereichen der medizinischen Diagnostik zahlreiche weitere Analysesysteme bekannt, welche grundsätzlich auch für tragbare Geräte geeignet sind. So beschreibt beispielsweise US 2004/0210280 A1 ein Pflaster-artiges System, welches für eine transdermale Therapie und Diagnose eingesetzt werden kann. Dabei wird unter anderem vorgeschlagen, dass das System selbstständig Flüssigkeitsproben aus der Haut sammelt und aufnimmt.

In A. Pais et al.: High-sensitivity, disposable lab-on-a-chip with thin-film organic electronics for fluorescence detection, Lab Chip, 2008, 8, 794-800 wird ein Einweg-lab-ona-chip-Testelement vorgeschlagen. Dieses basiert auf einer organischen lichtemittierenden Diode und einem organischen Photodetektor. Das Testelement ist als mikrofluidisches Testelement ausgestaltet und in der Lage, flüssige Proben mittels einer Fluoreszenzdetektion zu analysieren.

In DE 10 2004 048 864 A1 wird ein analytisches Testelement mit drahtloser Datenübertragung beschrieben, welches zur Bestimmung der Konzentration eines Analyten aus einer Körperflüssigkeit eingesetzt wird. Dabei wird vorgeschlagen, zumindest einen Teil der elektrischen Komponenten des Systems auf der Basis von Polymerelektronik auszugestalten.

In US 2006/020216 A1 wird ein tragbares Gesundheits-Management-Gerät beschrieben, welches insbesondere für eine Blutdruckmessung eingesetzt werden kann. Unter anderem wird dabei vorgeschlagen, die Bewegung des Bluts innerhalb eines Blutgefäßes mittels Lichtabsorption von transdermal eingestrahltem Licht zu messen.

Aus dem Bereich der medizinischen Therapeutik sind ebenfalls Verfahren und Vorrichtungen bekannt, bei welchen eine Hautoberfläche mit Licht einer Lichtquelle bestrahlt wird. So wird beispielsweise in I. Samuel: "Light fantastic", Materials World, August 2007, 28-30 eine Vorrichtung zur photodynamischen Therapie von Hautkrebserkrankungen beschrieben. Unter anderem wird dabei vorgeschlagen, ein selbstklebendes Pflaster mit einer organischen Leuchtdiode zu verwenden, um eine zwischen dem Pflaster und der Hautoberfläche angeordnete Creme zu bestrahlen. Durch die photochemische Reaktion wird dann das umgebende Krebsgewebe zerstört.

Allgemein wird zur Nierenfunktionsprüfung im Stand der Technik regelmäßig auf Inulin als Goldstandard zurückgegriffen. Die Inulin Messung erfolgt hierbei meistens enzymatisch, d.h. in einer entnommenen Serum- oder Urinprobe. Nicht-invasive Verfahren mit Fluoreszenz-markiertem Inulin lieferten uneindeutige Ergebnisse (WO2001/85799). Als Standard für Fluoreszenz basierte GFR Bestimmungen wurde FITC Sinistrin etabliert (WO2001/85799; Pill 2005, Anal Bioanal Chem 382: 59-64; Pill 2005, Europ J Medicinal Chem 40: 1056-1061), wobei auch hier die Messungen überwiegend in isolierten Proben erfolgten.

Diese letztgenannten, aus dem Stand der Technik bekannten Verfahren und Vorrichtungen sind jedoch allgemein vergleichsweise apparativ aufwändig. So erfordern Systeme, die auf einer Probensammlung basieren, wie beispielsweise das in der Veröffentlichung von A. Pais et al. Beschriebene System, in der Regel eine technisch aufwändige Mikrofluidik, welche in der Regel nur mittels entsprechender Mikrokanalstrukturen realisiert werden kann. Auch die anderen beschriebenen Systeme sind in der Regel technisch vergleichsweise aufwändig. Zudem ist keines der beschriebenen Systeme unmittelbar für eine Messung einer Nierenfunktion anwendbar.

Eine Aufgabe der vorliegenden Erfindung besteht somit darin, Vorrichtungen und Verfahren zur Bestimmung von Organfunktionen, insbesondere einer Nierenfunktion, bereitzustellen, welche die Nachteile bekannter Vorrichtungen und Verfahren vermeiden. Insbesondere soll eine einfach zu handhabende Vorrichtung bereitgestellt werden, welche auch ohne erhebliche Unterbrechung des Tagesablaufs der Patienten oder zumindest im Rahmen einer ambulanten Behandlung eine einfache, schnelle und dennoch zuverlässige Messung der Organfunktion ermöglicht. Diese Aufgabe wird durch die Erfindung mit den Merkmalen der unabhängigen Ansprüche gelöst. Vorteilhafte Weiterbildungen der Erfindung, welche einzeln oder in Kombination realisiert sein können, sind in den abhängigen Ansprüchen dargestellt.

Dabei werden ein Sensorpflaster, ein das Sensorpflaster umfassendes Sensorsystem, ein das Sensorpflaster oder das Sensorsystem umfassendes Kit, Verwendungen des Sensorpflasters, des Sensorsystems oder des Kits, sowie ein Verfahren zur Herstellung des Sensorpflasters, Verfahren zur transkutanen Messung einer Organfunktion vorgeschlagen und Verwendungen einer Fluoreszenz-markierten Indikatorsubstanz zur Herstellung eines Diagnostikums vorgeschlagen, welche optional auch kombiniert werden können. So kann beispielsweise das Verfahren zur transkutanen Messung einer Organfunktion unter Verwendung eines oder mehrerer der vorgeschlagenen Vorrichtungen durchgeführt werden, so dass für mögliche optionale Ausgestaltungen des Verfahrens auf die Beschreibung der jeweiligen Vorrichtungen verwiesen werden kann. Umgekehrt können die Vorrichtungen eingerichtet sein, um ein entsprechendes Verfahren durchzuführen. So können beispielsweise in den Vorrichtungen, beispielsweise dem Sensorpflaster, dem Sensorsystem oder dem Kit, ein oder mehrere Datenverarbeitungsgeräte vorgesehen sein, welche beispielsweise programmtechnisch eingerichtet sind, um Teilschritte des vorgegebenen Verfahrens in einer der im Folgenden beschriebenen Formen zu übernehmen.

Ein Grundgedanke der vorliegenden Erfindung besteht darin, bekannte optische Vorrichtungen und Verfahren zur Bestimmung der Organfunktion, beispielsweise der Nierenfunktion, durch Verwendung kleiner, integrierter Sensorpflaster zu verbessern. So wird in einer ersten Offenbarung ein Sensorpflaster zur transkutanen Messung einer Organfunktion, insbesondere einer Nierenfunktion, vorgeschlagen, welches beispielsweise für die Messung der renalen Clearance gemäß der obigen Beschreibung des Standes der Technik eingesetzt werden kann. Unter einem Pflaster wird im Rahmen der vorliegenden Erfindung allgemein ein medizinischer Artikel verstanden, welcher mindestens ein flexibles Trägerelement mit mindestens einer auf eine Körperoberfläche aufbringbaren, insbesondere aufklebbaren, Haftoberfläche aufweist. Dieses flexible Trägerelement kann beispielsweise einen Kunststoff, ein Textil, eine Keramik, ein Papier oder eine Kombination der genannten und/oder anderer Materialien umfassen. Das Sensorpflaster kann somit selbstklebend ausgestaltet sein und kann beispielsweise auf der Haftoberfläche ein oder mehrere Klebstoffe umfassen. In einem Lagerungszustand können die Klebstoffe beispielsweise durch eine oder mehrere Schutzfolien geschützt sein, welche beispielsweise abziehbar sind. Durch diese Haftoberfläche kann somit eine stoffschlüssige Verbindung zwischen dem Sensorpflaster und der Körperoberfläche möglich. Grundsätzlich sind jedoch, alternativ oder zusätzlich, auch andere Arten der Verbindungen zwischen der Haftoberfläche und der Körperoberfläche möglich, beispielsweise kraftschlüssige Verbindungen. So kann beispielsweise die Haftoberfläche mittels einer oder mehrerer Klemmvorrichtungen auf die Körperoberfläche aufgepresst werden, beispielsweise mittels einer Fingerklemme oder einer anderen Art mechanischer Vorrichtung, welche eine Anpresskraft zum Anpressen der Haftoberfläche auf die Körperoberfläche bereitstellen kann. Besonders bevorzugt ist jedoch die Verwendung selbstklebender Haftoberflächen. Grundsätzlich kann die Haftoberfläche also als selbstklebende Haftoberfläche ausgestaltet sein. Alternativ oder zusätzlich sind jedoch grundsätzlich auch Haftoberflächen denkbar, bei welchen nachträglich ein oder mehrere Klebstoffe aufgebracht werden können, um die Verbindung zu ermöglichen. Beispielsweise können mittels einer Klebstofftube hautverträgliche Klebstoffe auf die Haftoberfläche aufgebracht werden, um dann das Sensorpflaster auf die Körperoberfläche aufzukleben.

Als Körperoberfläche kommen dabei grundsätzlich beliebige Oberflächen eines Körpers eines menschlichen oder tierischen Patienten in Betracht. Als Beispiele sind Hautoberflächen, Oberflächen von Fingernägeln oder Zehennägeln oder andere Oberflächen zu nennen, insbesondere Oberflächen, welche der Atmosphäre ausgesetzt sind. Allgemein wird dabei im Rahmen der vorliegenden Erfindung der Begriff "Patient" für einen Menschen oder ein Tier verwendet, an welchem eine oder mehrere der vorgeschlagenen Vorrichtungen und/oder Verfahren eingesetzt werden sollen, unabhängig davon, ob dieser Menschen oder dieses Tier gesund oder krank ist.

Weiterhin umfasst das Sensorpflaster mindestens eine Strahlenquelle. Unter einer Strahlenquelle wird dabei eine beliebige Vorrichtung verstanden, welche in der Lage ist, Strahlung zu emittieren. Dabei kann es sich insbesondere um elektromagnetische Strahlung handeln, beispielsweise Licht im sichtbaren und/oder infraroten und/oder ultravioletten Spektralbereich und/oder um Gammastrahlung. Alternativ oder zusätzlich sind jedoch grundsätzlich auch andere Arten von Strahlen einsetzbar, beispielsweise Partikelstrahlen. In diesem Zusammenhang sind beispielsweise Alphastrahlen und/oder Betastrahlen zu erwähnen. Die Strahlenquelle ist entsprechend ausgestaltet, um Strahlung der genannten Art zu erzeugen. Ohne Beschränkung der möglichen weiteren Ausgestaltungen der Strahlung wird die Strahlung im Folgenden allgemein als "Licht" bezeichnet, die Handhabung der Strahlung als "Optik", und die Strahlenquelle wird insbesondere unter Bezugnahme auf eine Lichtquelle beschrieben. Auch andere Ausgestaltungen der Strahlenquelle sind jedoch grundsätzlich möglich, und es ist beispielsweise auch möglich, verschiedene Arten von Strahlenquellen zu kombinieren.

Die Strahlenquelle kann insbesondere integraler Bestandteil des Pflasters sein, beispielsweise im Rahmen eines Schichtaufbaus des Sensorpflasters. Die Strahlenquelle ist also eingerichtet, um unmittelbar innerhalb des Sensorpflasters mindestens ein Abfragelicht zu erzeugen, im Gegensatz zu einer externen Erzeugung des Abfragelichts. Insofern unterscheidet sich das Sensorpflaster beispielsweise von der faseroptischen Konstruktion der DE 100 23 051 A1, bei welcher eine externe Lichtquelle verwendet wird. Anstelle einer einzelnen Lichtquelle können auch mehrer Lichtquellen verwendet werden, beispielsweise redundante Lichtquellen zur Emission ein und derselben Wellenlänge, und/oder mehrere verschiedene Lichtquellen zur Emission unterschiedlicher Wellenlängen. Allgemein soll die mindestens eine Lichtquelle eingerichtet sein, um die Körperoberfläche mit mindestens einem Abfragelicht zu bestrahlen.

Unter einem Abfragelicht wird dabei im Rahmen der vorliegenden Erfindung ein für die Detektion der Indikatorsubstanz verwendbares Licht im Sinne der obigen Definition verstanden, welches die Indikatorsubstanz im Inneren eines Körpergewebes und/oder einer Körperflüssigkeit, beispielsweise bei variabler Eindringtiefe, zu einer wahrnehmbaren Antwort, insbesondere einer optisch wahrnehmbaren Antwort, anregt. Diese Anregung kann beispielsweise derart erfolgen, dass eine Lumineszenz, insbesondere eine Fluoreszenz und/oder eine Phosphoreszenz, in der Indikatorsubstanz angeregt wird. Alternativ oder zusätzlich kann jedoch auch eine andere Art von Anregung erfolgen, beispielsweise eine Streuung des Lichts bei gleicher oder verschobener Wellenlänge. Allgemein wird bei dieser Antwort der Indikatorsubstanz mindestens ein Antwortlicht generiert.

Das Abfragelicht soll dabei derart eingerichtet sein, dass die gewünschte Antwort in der Indikatorsubstanz gezielt angeregt wird. Dementsprechend können beispielsweise eine Wellenlänge und/oder ein Wellenlängenbereich des Abfragelichts und/oder eine andere Eigenschaft des Abfragelichts angepasst sein. Dies kann beispielsweise unmittelbar durch die Strahlenquelle erfolgen, indem diese Strahlenquelle beispielsweise bereits Abfragelicht der gewünschten Wellenlänge und/oder des gewünschten Wellenlängenbereichs bereitstellt und/oder indem zusätzlich mindestens ein Anregungsfilter verwendet wird, welcher aus einem Primärlicht der Lichtquelle das gewünschte Abfragelicht herausfiltert. Besonders bevorzugt ist es dabei, wenn das Sensorpflaster eingerichtet ist, um Fluoreszenzmessungen an der Indikatorsubstanz vorzunehmen. Dementsprechend kann das Abfragelicht auf einen Anregungsbereich dieser Fluoreszenz der Indikatorsubstanz angepasst sein. Wird beispielsweise eine Fluoreszenz von FITC angeregt, so kann Abfragelicht im spektralen Bereich um 480 nm verwendet werden, beispielsweise Abfragelicht, welches im Bereich zwischen 470 nm und 490 nm eine wahrnehmbare Intensität aufweist.

Das Sensorpflaster umfasst weiterhin mindestens einen Detektor, welcher eingerichtet ist, um mindestens ein aus Richtung der Körperoberfläche eingestrahltes Antwortlicht zu erfassen. Wiederum kann es sich bei dem Antwortlicht um Licht im Sinne der obigen Definition handeln. Auch der Detektor kann wiederum integraler Bestandteil des Sensorpflasters sein. Der Detektor ist somit Teil des Sensorpflasters, so dass eine Detektion des Antwortlichts unmittelbar innerhalb des Pflasters erfolgt, im Gegensatz beispielsweise zu der faseroptischen Konstruktion der DE 100 23 051 A1, bei welcher ein externer Detektor verwendet werden muss.

Das Antwortlicht stellt die optische Antwort der Indikatorsubstanz auf die Einstrahlung des Abfragelichts dar. Dementsprechend können der Detektor und/oder der Detektor in Zusammenwirkung mit mindestens einem Antwortfilter eingerichtet sein, um gezielt im spektralen Bereich des Antwortlichts zu detektieren. Dabei können der Detektor und/oder der Detektor in Zusammenwirkung mit dem mindestens einen Antwortfilter eingerichtet sein, um Licht außerhalb des spektralen Bereichs des Antwortlichts zu unterdrücken. Insbesondere können der Detektor und/oder der Detektor in Zusammenwirkung mit dem mindestens einen Antwortfilter eingerichtet sein, um Abfragelicht zu unterdrücken. Das Abfragelicht und das Antwortlicht können insbesondere spektral unterschiedlich bzw. spektral gegeneinander verschoben ausgestaltet sein, also verschieden bezüglich ihrer spektralen Intensitätsverteilung. Insbesondere kann das Antwortlicht im Vergleich zum Abfragelicht zu längeren Wellenlängen hin verschoben sein, was beispielsweise bei einer Fluoreszenzmessung in der Regel der Fall ist. Beispielsweise kann die spektrale Verschiebung einer Peakwellenlänge des Antwortlichts gegenüber einer Peakwellenlänge des Abfragelichts zwischen 10 nm und 100 nm, insbesondere zwischen 30 nm und 50 nm und besonders bei ca. 40 nm liegen. Der Detektor und/oder der Detektor in Zusammenwirkung mit dem mindestens einen Antwortfilter können dementsprechend eingerichtet sein, um derartiges Antwortlicht zu detektieren. Bei Verwendung von FITC können beispielsweise der Detektor und/oder der Detektor in Zusammenwirkung mit dem mindestens einen Antwortfilter eingerichtet sein, um Antwortlicht zu detektieren, welches im Bereich zwischen 510 nm und 530 nm, insbesondere bei 520 nm, eine messbare Intensität aufweist.

Die mindestens eine Strahlenquelle, insbesondere die mindestens eine Lichtquelle, und der mindestens eine Detektor sind eingerichtet, um die Körperoberfläche mit dem Abfragelicht zu bestrahlen und um mindestens ein aus Richtung der Körperoberfläche eingestrahltes Antwortlicht zu erfassen. Die Strahlenquelle und der Detektor sind also derart optisch mit der Körperoberfläche verbunden, dass durch die Körperoberfläche hindurch, beispielsweise transkutan, das Abfragelicht in das Körpergewebe bzw. die Körperflüssigkeit eingestrahlt werden kann und dass, ebenfalls durch die Körperoberfläche hindurch, beispielsweise wiederum transkutan, das Antwortlicht aus dem Körpergewebe bzw. der Körperflüssigkeit von dem Detektor aufgenommen werden kann. Damit unterscheidet sich das vorgeschlagene Sensorpflaster beispielsweise von Lab-on-a-Chip-Systemen, insbesondere von mikrofluidischen Systemen, welche eine Probennahme und in der Regel eine aufwändige Mikrokanalstruktur erfordern.

Die erfindungsgemäße transkutane Messung kann beispielsweise dadurch erfolgen, dass die Strahlenquelle und/oder der Detektor unmittelbar und flächig auf der Körperoberfläche aufliegen. Beispielsweise kann die Strahlenquelle eine Emissionsoberfläche umfassen, welche direkt oder unter Zwischenschaltung einer oder mehrerer transparenter Schichten auf die Körperoberfläche aufgelegt werden kann. Entsprechend kann der mindestens eine Detektor mindestens eine Sensoroberfläche umfassen, welche beispielsweise direkt oder unter Zwischenschaltung einer oder mehrerer transparenter Schichten auf die Körperoberfläche aufgebracht werden kann und über welche das Abfragelicht emittiert und das Antwortlicht aufgenommen werden kann.

Grundsätzlich lassen sich zahlreiche Arten von Strahlenquellen für das vorgeschlagene Sensorpflaster einsetzen. Es ist dabei besonders bevorzugt, wenn die mindestens eine Strahlenquelle als großflächige Strahlenquelle ausgestaltet ist, also als Strahlenquelle mit einer eine Strahlung emittierenden Fläche, beispielsweise einer lichtemittierenden Fläche, im Gegensatz beispielsweise zu Punktlichtquellen bzw. Punktstrahlenquellen. Beispielsweise lassen sich großflächige Lichtquellen mit einer lichtemittierenden Fläche von mindestens 0,2 cm², vorzugsweise mindestens 0,5 cm² und besonders bevorzugt 1 cm² oder mehr an lichtemittierender Fläche einsetzen.

Besonders bevorzugt ist es, wenn die mindestens eine Strahlenquelle mindestens eine Lichtquelle mit einem organischen lichtemittierenden Material umfasst, insbesondere eine organische lichtemittierende Diode (OLED). Unter einem organischen lichtemittierenden Material kann dabei grundsätzlich ein beliebiges organisches Material natürlichen und/oder synthetischen Ursprungs verstanden werden, welches in der Lage ist, Licht zu emittieren. Somit fallen unter diesen Begriff der OLED beispielsweise auch bio-organische lichtemittierende Dioden. Die Erzeugung des Lichts in dem organischen Material kann dabei auf verschiedenen Mechanismen basieren. So kann beispielsweise eine Elektrolumineszenz genutzt werden, also eine Anregung des organischen Materials zur Lichtemission mittels eines elektrischen Stroms. Auch andere Mechanismen sind jedoch grundsätzlich möglich, beispielsweise eine Biolumineszenz oder andere Mechanismen. Auch eine Kombination von verschiedenen Mechanismen zur Lichterzeugung ist denkbar.

Neben den organischen lichtemittierenden Materialien und den entsprechenden lichtemittierenden Schichten können weitere Materialien bzw. Funktionsschichten vorgesehen sein, beispielsweise Ladungsträger-Transportschichten, Barriereschichten oder ähnliche Materialien und Schichten. Dabei können rein organische Bauelemente verwendet werden, also Bauelemente, welche ausschließlich organische lichtemittierende Materialien und organische Funktionsschichten umfassen, oder es können auch Hybrid-Bauelemente eingesetzt werden, also Bauelemente, welche sowohl anorganische als auch organische lichtemittierende Materialien bzw. Funktionsschichten umfassen. Beides soll im Folgenden von dem Begriff einer organischen lichtemittierenden Diode umfasst sein.

Bezüglich des Aufbaus organischer lichtemittierender Dioden kann beispielsweise auf die aus dem Stand der Technik bekannten Aufbauten verwiesen werden. Beispielsweise kann auf die in der oben zitierten Veröffentlichung von A. Pais et al. beschriebenen organischen lichtemittierenden Dioden verwiesen werden oder den in dieser Druckschrift zitierten Stand der Technik zu OLEDs.

Als organische Materialien lassen sich beispielsweise niedermolekulare organische Materialien einsetzen, also beispielsweise Monomere und/oder Oligomere. Als Beispiel derartiger niedermolekularer Substanzen kann ebenfalls auf die in der oben genannten Veröffentlichung von A. Pais et al. verwendeten Substanzen verwiesen werden. Alternativ oder zusätzlich lassen sich auch Polymermaterialien einsetzen, beispielsweise konjugierte Polymere. Typische Polymermaterialien dieser Art sind beispielsweise Fluorene oder Polyphenylenvinylen-Derivate (PPVs) zu nennen. Die organischen Materialien können, je nach ihren Verarbeitungseigenschaften, beispielsweise aus der Gasphase abgeschieden werden oder auch aus der flüssigen Phase, beispielsweise mittels eines Aufschleuderverfahrens oder eines Druckprozesses. Organische Leuchtdioden zeichnen sich dadurch aus, dass sich mit dieser Technik großflächige, homogen emittierende Lichtquellen herstellen lassen, mittels derer ein großer Bereich der Körperoberfläche bestrahlbar ist.

Alternativ oder zusätzlich zu der vollständigen oder teilweisen Ausgestaltung der Lichtquelle als Lichtquelle mit organischem lichtemittierendem Material kann auch der mindestens eine Detektor ganz oder teilweise als zumindest teilweise organischer Detektor ausgestaltet sein. So kann der mindestens eine Detektor mindestens einen Detektor mit mindestens einem organischen halbleitenden Material umfassen, insbesondere einen organischen Photodetektor (OPD).

Auch bezüglich organischer Photodetektoren, welche beispielsweise ganz oder teilweise als organische Solarzelle und/oder als organische Photodiode ausgestaltet sein können, kann weitgehend auf die Literatur verwiesen werden. So kann beispielsweise bezüglich möglicher Ausgestaltungen des organischen Photodetektors wiederum auf die oben zitierte Veröffentlichung von A. Pais et al. verwiesen werden. Wiederum lassen sich vollständig organische Bauelemente einsetzen, oder es lassen sich auch Hybridbauelemente einsetzen, welche eine Kombination aus organischen und anorganischen Materialien bzw. Funktionsschichten umfassen. Wiederum lassen sich niedermolekulare organische Substanzen einsetzen, also Monomere oder Oligomere, oder, alternativ oder zusätzlich, wiederum auch Polymere. Auch bezüglich möglicher Abscheideverfahren bzw. Erzeugungsverfahren der organischen Bauelemente kann zumindest weitgehend auf die obige Beschreibung verwiesen werden.

Analog zu den oben beschriebenen Vorteilen organischer Leuchtdioden weisen auch OPDs ähnliche Vorteile auf. So lassen sich in dieser Technologie großflächige, dünne Photodetektoren herstellen, welche, ähnlich wie OLEDs, unmittelbar in die Sensorpflaster integrierbar sind. Beispielsweise lässt sich insgesamt eine Schichttechnologie einsetzen, bei welcher die Sensorpflaster Schicht für Schicht aufgebaut werden. Auf diese Weise lassen sich in Schichtbauweise Sensorpflaster herstellen, die mindestens zwei verschiedene Schichtebenen aufweisen. Eine dieser Schichtebenen kann beispielsweise das mindestens eine flexible Trägerelement sein, und andere dieser Schichtebenen können beispielsweise elektronische Bauelemente umfassen, beispielsweise den Detektor und/oder die Strahlenquelle.

Neben dem mindestens einen Detektor und der mindestens einen Strahlenquelle kann das Sensorpflaster weitere Elemente umfassen. So kann das Sensorpflaster beispielsweise mindestens eine Schnittstelle zum Austausch von Daten umfassen. Bei diesen Daten kann es sich beispielsweise um Messergebnisse handeln, beispielsweise Intensitäten des Antwortlichts, welches von dem Detektor erfasst wurde. Auch bereits teilweise verarbeitete Daten, beispielsweise gefilterte oder teilweise oder vollständig ausgewertete Daten, lassen sich über diese Schnittstelle übertragen. Die Schnittstelle kann insbesondere als drahtlose Schnittstelle ausgestaltet sein und kann insbesondere eine Hochfrequenzspule umfassen. Insofern kann eine aus dem Stand der Technik bekannte Transpondertechnologie beispielsweise auch eingesetzt werden, um eine Messung mittels des Sensorpflasters zu initiieren und/oder Messdaten von dem Sensorpflaster abzufragen. Zu diesem Zweck können entsprechende Hochfrequenz-Lesegeräte eingesetzt werden, wie sie beispielsweise aus der RFID-Technologie (Hochfrequenz-Identifikationsetiketten-Technologie) bekannt sind.

Weiterhin kann das Sensorpflaster mindestens eine Ansteuerelektronik umfassen. Diese Ansteuerelektronik kann beispielsweise ausgestaltet sein, um die mindestens eine Strahlenquelle und den mindestens einen Detektor anzusteuern, beispielsweise um eine Emission des Abfragelichts zu starten und/oder eine Detektion des Antwortlichts zu initiieren. Zu diesem Zweck kann die Ansteuerelektronik beispielsweise entsprechende Treiber für den Detektor und/oder die Strahlenquelle umfassen. Auch ein Timing für eine Messung kann vorgegeben sein, so dass beispielsweise die Ansteuerelektronik ein bestimmtes Zeitschema für die Strahlenquelle und/oder den Detektor vorgeben kann, welches eine zeitliche Abfolge der Emission des Abfragelichts und der Detektion des Antwortlichts ermöglicht. Beispielsweise kann die Ansteuerelektronik eingerichtet sein, um eine zeitaufgelöste Messung des Sensorpflasters durchzuführen bzw. zu steuern. Eine Messung umfasst dabei die Emissionen mindestens eines Abfragelichts, insbesondere mindestens eines Pulses des Abfragelichts, und die Detektion mindestens eines Antwortlichts, insbesondere mindestens eines Pulses des Antwortlichts. Unter einer zeitaufgelösten Messung kann dementsprechend eine Messung verstanden werden, bei welcher zusätzlich auch eine Zeit der Detektion des Antwortlichts eine Rolle spielt bzw. registriert wird. So können beispielsweise zu jedem Wert des Antwortlichts auch die entsprechenden Zeitpunkte der Aufnahme dieses Werts registriert werden und/oder es kann nur eine Aufnahme des Antwortlichts zu bestimmten Zeitpunkten erfolgen (Gating). Auf diese Weise lassen sich mittels zeitaufgelöster Messungen beispielsweise Informationen über eine Tiefe gewinnen, aus welcher das jeweilige Antwortlicht stammt, beispielsweise über Laufzeitenmessungen. Alternativ oder zusätzlich lassen sich auch komplexe Messschemata einsetzen, bei welchen beispielsweise zu einem vorgegebenen Zeitpunkt nach der Anregung durch das Abfragelicht das Antwortlicht erfasst wird.

Weiterhin kann die Ansteuerelektronik, ebenfalls alternativ oder zusätzlich, auch eingerichtet sein, um eine teilweise oder vollständige Verarbeitung der Messergebnisse durchzuführen. Insbesondere können dabei die von dem mindestens einen Detektor aufgenommenen Signale verarbeitet werden sowie optional zusätzliche Informationen, wie beispielsweise Zeitinformationen, beispielsweise die Zeitpunkte, zu welchen die Messsignale des Detektors aufgenommen wurden. Bei den Messwerten bzw. Messsignalen des Detektors kann es sich beispielsweise um Intensitäten des Antwortlichts und/oder um mit diesen Intensitäten korrelierende Signale elektrischer Art handeln. In diesem Fall kann beispielsweise eine vollständige oder teilweise Verarbeitung dieser Signale erfolgen, so dass beispielsweise bereits eine Filterung, eine Glättung, eine Mittelwertbildung oder Ähnliches in der Ansteuerelektronik erfolgt. Alternativ oder zusätzlich kann auch bereits zumindest teilweise eine Auswertung dieser Signale erfolgen, beispielsweise eine Ermittlung einer Kurvenform und/oder einer Halbwertszeit und/oder eine Ermittlung einer diesen Signalen entsprechenden Konzentration der Indikatorsubstanz.

Auch eine teilweise oder vollständige Speicherung der Informationen in dem Sensorpflaster, insbesondere in der Ansteuerelektronik ist denkbar. Diese Informationen können beispielsweise ein oder mehrere Detektorsignale beziehungsweise daraus abgeleitete Informationen umfassen, Zeitinformationen, Informationen über das Abfragelicht, beispielsweise eine Intensität des Abfragelichts, oder Kombinationen der genannten Informationen und/oder weiterer Informationen. Zur Speicherung der Informationen kann das Sensorpflaster, insbesondere die Ansteuerelektronik, beispielsweise einen oder mehrere Datenspeicher umfassen, insbesondere flüchtige und/oder nicht-flüchtige Datenspeicher. Allgemein kann die Ansteuerelektronik ganz oder teilweise unter Verwendung elektrischer Komponenten ausgestaltet sein, wobei auch ein oder mehrere Datenverarbeitungsgeräte, beispielsweise Mikroprozessoren und/oder ASICs verwendet werden können.

Die Ansteuerelektronik kann auch ganz oder teilweise als organische Elektronik ausgestaltet sein. So kann die Ansteuerelektronik beispielsweise mindestens ein organisches Bauelement umfassen, also ein Bauelement, welches mindestens ein organisches Material, insbesondere ein aktives organisches Material, umfasst. Beispielsweise kann es sich dabei um organische Leiter und/oder Halbleiter handeln. Das organische Bauelement kann beispielsweise einen organischen Feldeffekttransistor umfassen oder einfach eine organische Leiterbahn.

Derartige organische Bauelemente sind beispielsweise in Form von Polymerelektronik aus DE 10 2004 048 864 A1 bekannt. Beispielsweise lassen sich organische Feldeffekttransistoren unter Verwendung organischer Halbleitermaterialien herstellen, welche Bestandteil der Ansteuerelektronik sein können. Auch einfachere organische Bauelemente können umfasst sein, wie beispielsweise einfache Leiterbahnen und/oder Anschlusskontakte, welche ein organisches leitendes Material umfassen, beispielsweise ein leitfähiges Polymer. Der Vorteil derartiger vollständig oder teilweise in organischer Technik aufgebauter Ansteuerelektroniken liegt wiederum darin, dass derartige Ansteuerelektroniken flach, klein und kostengünstig hergestellt werden können, so dass diese auch in Einwegartikeln wie beispielsweise Pflastern eingesetzt werden können. Wiederum lassen sich einfache und kostengünstige Schichtbauweisen für die Erzeugung der Ansteuerelektronik einsetzen, beispielsweise Drucktechniken oder Ähnliches. Allgemein lässt sich das Sensorpflaster vorzugsweise in einer Rolle-zu-Rolle-Technik herstellen, bei welcher zahlreiche Sensorpflaster als Bandware hergestellt werden.

Allgemein ist es besonders bevorzugt, wenn die Ansteuerelektronik robust und unanfällig gegenüber Fehlern ausgestaltet ist. So kann beispielsweise die Ansteuerelektronik ausgestaltet sein, um einen Abgleich und/oder eine Kalibration zu ermöglichen. Beispielsweise können in der Ansteuerelektronik entsprechende Abgleichelemente vorgesehen sein, welche einen Abgleich ermöglichen. Beispielsweise kann es sich dabei um einstellbare Abgleichelemente handeln und/oder um Elemente, welcher ein Trimming erlauben. Letzteres kann beispielsweise durch Abgleichelemente erfolgen, welche mittelst eines geeigneten Trimming-Prozesses, beispielsweise eines mechanischen Trimming-Verfahrens und/oder eines Laser-Trimmings auf die gewünschten Eigenschaften einstellbar sind. Auch ein Trimming auf variable Eigenschaften ist grundsätzlich möglich, beispielsweise ein Trimming auf eine variable Wellenlänge des Abfragelichts und/oder des Antwortlichts. Beispielsweise kann mittels eines derartigen Trimming-Prozesses eine Länge eines Abgleichelements eingestellt werden.

Weiterhin kann die Ansteuerelektronik auch auf andere Weise ausgestaltet sein, um wiederholbare Messsituationen zu ermöglichen. So kann die Ansteuerelektronik beispielsweise redundant ausgestaltet sein und ein oder mehrere Elemente mehrfach enthalten, beispielsweise um einen Ausfall und/oder eine Fehlfunktion eines dieser Elemente zu kompensieren. Weiterhin lassen sich auch kalibrierte Bauelemente einsetzen, beispielsweise kalibrierte Verstärker, kalibrierte analog-digital-Wandler, kalibrierte Strahlenquellen, kalibrierte Detektoren oder ähnliches. Weiterhin lassen sich fehlertolerante Schaltkreise, redundante Schaltkreise und/oder kompensatorische Schaltkreise einsetzen, welche eine Funktionsfähigkeit sicherstellen können. Weiterhin lassen sich auch Testschaltkreise implementieren, welche beispielsweise während einer Kalibration erforderliche Parameter intern speichern können und es ermöglichen, dass sich das Sensorpflaster, insbesondere die Ansteuerelektronik, selbst rekonfigurieren kann. Auf diese Weise lassen sich beispielsweise defekte Elemente umgehen, es lassen sich Lastwiderstände einstellen oder ähnliches.

Weiterhin kann das vorgeschlagene Sensorpflaster mindestens ein Filterelement umfassen. Dieses Filterelement kann sowohl im Strahlengang des Abfragelichts als auch im Strahlengang des Antwortlichts zum Einsatz kommen, wobei beide Möglichkeiten auch in Kombination realisierbar sind. So kann beispielsweise mindestens ein Filterelement im Strahlengang des Antwortlichts zum Einsatz kommen, also mindestens ein Antwortfilter, und/oder mindestens ein Filterelement im Strahlengang des Abfragelichts, also mindestens ein Anregungsfilter. Der mindestens eine Antwortfilter und der mindestens eine Anregungsfilter können dabei unterschiedliche spektrale Eigenschaften aufweisen, beispielsweise unterschiedliche Peaktransmissionen. Der mindestens eine Anregungsfilter und der mindestens eine Abfragefilter können als separate Bauelemente ausgestaltet sein oder können auch ganz oder teilweise als gemeinsames Bauelement ausgestaltet sein. Weiterhin ist auch eine Ausgestaltung, bei welcher lediglich in einem der genannten Strahlengänge ein Filterelement vorgesehen ist, denkbar.

Das mindestens eine Filterelement kann beispielsweise genutzt werden, um das Abfragelicht spektral von dem Antwortlicht zu trennen. Beispielsweise können das Abfragelicht und das Antwortlicht spektral unterschiedlich ausgestaltet sein, beispielsweise spektral zumindest teilweise gegeneinander verschoben. Auf diese Weise kann beispielsweise vor dem Detektor ein Filterelement eingesetzt werden, welches zumindest teilweise verhindert, dass Abfragelicht in den Detektor gelangt und dort einen störenden Messuntergrund und/oder Hintergrund bildet. Umgekehrt kann beispielsweise, alternativ oder zusätzlich, vor der Strahlenquelle ein weiteres Filterelement eingesetzt werden, welches aus dem Spektrum der Strahlenquelle, welches beispielsweise breitbandig ausgestaltet sein kann, lediglich einen bestimmten Spektralbereich für das Abfragelicht herausfiltert. Verschiedene Kombinationen sind denkbar.

Als Filterelement können grundsätzlich sämtliche Filterelemente mit spektral trennenden Eigenschaften zum Einsatz kommen. Als Beispiel seien hier Interferenzfilter, dichroitische Spiegel, Absorptionsfilter oder Ähnliches genannt. Besonders bevorzugt ist es, wenn das mindestens eine Filterelement mindestens eine Filterfolie umfasst, also ein dünnes flexibles Element. Diese Filterfolie kann beispielsweise in Schichttechnologie auf die übrigen Schichten aufgeklebt und/oder aufgedruckt werden. Auch eine Kombination mehrerer Filterfolien ist denkbar. Das mindestens eine Filterelement kann auch ganz oder teilweise in der Strahlenquelle und/oder dem Detektor integriert sein. Beispielsweise kann eine Strahlenquelle mit integriertem Anregungsfilter verwendet werden und/oder einen Detektor mit integriertem Antwortfilter.

Weiterhin kann das Sensorpflaster mindestens ein Abbildungssystem umfassen, also ein System mit mindestens einem das Licht, also das Abfragelicht und/oder das Antwortlicht, brechenden Eigenschaften. Auf diese Weise kann beispielsweise das Abfragelicht auf einen bestimmten Körperbereich fokussiert werden und/oder das Antwortlicht von einem Körperbereich kann auf den Detektor fokussiert werden. Um eine möglichst einfache, Platz sparende und kostengünstige Ausgestaltung des Abbildungssystems zu ermöglichen, ist es besonders bevorzugt, wenn dieses Abbildungssystem mindestens eine Fresnellinse umfasst. Derartige Linsen können beispielsweise in Druck- und/oder Prägetechnik hergestellt werden, beispielsweise indem die entsprechenden Fresnel-Strukturen in eine transparente Kunststofffolie eingeprägt werden. Die derart geprägte Folie kann zuvor oder nach dieser Behandlung auf die übrigen Schichten des Sensorpflasters aufgebracht werden, beispielsweise durch Aufkleben.

Weiterhin ist es besonders bevorzugt, wenn das Sensorpflaster mindestens einen elektrischen Energiespeicher umfasst. Dieser mindestens eine elektrische Energiespeicher ermöglicht es, dass das Sensorpflaster autark betrieben werden kann, ohne eine drahtlose oder drahtgebundene Verbindung zur Übertragung elektrischer Energie mit einer anderen Komponente herstellen zu müssen. Derartige Verbindungen sind jedoch grundsätzlich alternativ oder zusätzlich ebenfalls möglich. Der mindestens eine elektrische Energiespeicher sollte dabei möglichst flach und vorzugsweise flexibel ausgestaltet sein. Dementsprechend kann dieser mindestens eine elektrische Energiespeicher beispielsweise eine Polymer-Batterie umfassen. Verschiedene Ausgestaltungen sind denkbar.

Alternativ oder zusätzlich zur Verwendung eines elektrischen Energiespeichers ist jedoch auch eine Bereitstellung der für den Betrieb des Sensorpflasters benötigten elektrischen Energie auf andere Weise denkbar. So kann beispielsweise elektrische Energie von außen eingestrahlt werden, wie dies beispielsweise bei RFID-Etiketten in der Regel erfolgt. Wiederum alternativ oder zusätzlich kann auch auf andere Weise Energie der Umgebung entzogen werden, beispielsweise in Form von Wärme und/oder Licht. Derartige Vorrichtungen, welche der Umgebung des Sensorpflasters Energie in irgendeiner Form entziehen und die Energie als elektrisch nutzbare Energie für den Betrieb des Sensorpflasters bereitstellen, werden im Folgenden als Energieerzeugungsvorrichtung bezeichnet. Entsprechend kann das Sensorpflaster optional eine oder mehrere derartiger Energieerzeugungsvorrichtungen umfassen. So kann beispielsweise das Sensorpflaster mindestens eine der folgenden Vorrichtungen enthalten: ein Thermoelement, insbesondere ein Seebeck-Element und/oder ein Peltierelement, zur Umwandlung von Wärmeenergie in elektrische Energie; eine Solarzelle zur Umwandlung von Licht in elektrische Energie; ein Piezoelement zur Umwandlung mechanischer Energie, insbesondere von Vibrationen, in elektrische Energie. Auch Kombinationen der genannten und/oder anderer Arten von Energieerzeugungsvorrichtungen sind einsetzbar.

Wird beispielsweise eine Solarzelle als Energieerzeugungsvorrichtung und/oder als Bestandteil dieser Energieerzeugungsvorrichtung eingesetzt, so kann diese Solarzelle beispielsweise wiederum ganz oder teilweise als organische Solarzelle aufgebaut sein. Bezüglich möglicher Ausgestaltungen kann weitgehend auf die Beschreibung des Detektors verwiesen werden. Im Gegensatz zum Detektor ist die Solarzelle dann jedoch derart angeordnet, dass eine aktive Fläche der Solarzelle nicht der Körperoberfläche, beispielsweise der Hautoberfläche, zuweist, sondern einer Richtung, aus welcher in einem Zustand, in welchem das Sensorpflaster auf der Körperoberfläche aufgebracht ist, in der Regel eine Lichteinstrahlung von Umgebungslicht, insbesondere eine Sonneneinstrahlung, zu erwarten ist. So kann beispielsweise das Sensorpflaster auf einer von der aktiven Fläche der Strahlenquelle und/oder des Detektors abgewandten Seite des Trägerelements eine oder mehrere Solarzellen, insbesondere organische Solarzellen, umfassen, welche elektrische Energie an das auf die Körperoberfläche aufgebrachte Sensorpflaster bereitstellen können. Diese Bereitstellung kann unmittelbar an den Detektor, an die Strahlenquelle, an die Ansteuerelektronik oder an andere elektrische Komponenten des Sensorpflasters erfolgen, oder es kann eine Zwischenspeicherung der elektrischen Energie erfolgen, beispielsweise wiederum in einem oder mehreren elektrischen Energiespeichern, insbesondere Polymerbatterien. Verschiedene Ausgestaltungen sind denkbar.

Wie oben dargestellt, ist das Sensorpflaster insgesamt vorzugsweise ganz oder teilweise in Schichtbauweise hergestellt und umfasst mindestens zwei verschiedene Schichtebenen. Eine derartige Schichtbauweise ermöglicht einen integrierten Aufbau hoher Integrationsdichte. Gleichzeitig lassen sich kostengünstige Techniken einsetzen. Insbesondere lassen sich eines oder mehrere der folgenden Elemente ganz oder teilweise in einer Schichtbauweise herstellen: eine die mindestens eine Strahlenquelle und den mindestens einen Detektor umfassende optische Einheit; eine die Ansteuerelektronik umfassende elektronische Einheit; eine die Schnittstelle umfassende Kommunikationseinheit; ein die optische Einheit, die elektronische Einheit und die Kommunikationseinheit umfassendes Sensormodul. Für die Erzeugung eines Schichtaufbaus sind verschiedene Techniken einsetzbar, beispielsweise Laminiertechniken, Prägetechniken, Klebetechniken, Drucktechniken oder Kombinationen der genannten und/oder anderer Techniken. Besonders bevorzugt ist es, wenn die Strahlenquelle und/oder der Detektor zumindest teilweise mittels einer Drucktechnik auf das Trägerelement aufgebracht werden. Entsprechend wird ein derartiges Verfahren zur Herstellung des Sensorpflasters vorgeschlagen. Auch andere Komponenten des Sensorpflasters, beispielsweise eine oder mehrere der oben genannten Komponenten, lassen sich mittels der Drucktechnik herstellen. Alternativ oder zusätzlich zur Drucktechnik, welche beispielsweise einen Offset-Druck, einen Siebdruck, einen Inkjet-Druck, einen Tampon-Druck, einen Flexo-Druck oder eine Kombination der genannten und/oder anderer Druckarten umfassen kann, lassen sich auch andere Schichttechnologien einsetzen, beispielsweise Stempeltechniken, Prägetechniken oder Ähnliches. Insbesondere lässt sich auch die Polymerelektronik, welche optional beispielsweise in der Ansteuerelektronik umfasst sein kann, auf diese Weise herstellen.

Neben dem Sensorpflaster wird weiterhin ein Sensorsystem zur transkutanen Messung einer Organfunktion, insbesondere einer Nierenfunktion, vorgeschlagen. Das Sensorsystem umfasst mindestens ein Sensorpflaster gemäß einer oder mehreren der oben beschriebenen Formen. Weiterhin umfasst das Sensorsystem mindestens ein Lesegerät, welches eingerichtet ist, um mit dem Sensorpflaster zusammenzuwirken, wobei auch eine Zusammenwirkung mit mehreren Sensorpflastern möglich ist. Unter einer Zusammenwirkung lässt sich dabei allgemein eine funktionelle Wechselwirkung verstehen, bei welcher, zum Zweck der transkutanen Messung der Organfunktion, Steuersignale und/oder Informationen zwischen dem Lesegerät und dem mindestens einen Sensorpflaster ausgetauscht werden. Insbesondere kann das Lesegerät eingerichtet sein, um eine Messung der Organfunktion mittels des Sensorpflasters zu initiieren. Alternativ oder zusätzlich kann das Lesegerät auch beispielsweise eingerichtet sein, um Informationen von dem Sensorpflaster zu empfangen, beispielsweise die oben dargestellten Informationen. Das Lesegerät kann als Standgerät oder, vorzugsweise, als tragbares Gerät ausgestaltet sein. Zur Initiierung der Organfunktion kann beispielsweise mindestens eine Schnittstelle vorhanden sein, beispielsweise mindestens eine drahtlose und/oder eine drahtgebundene Schnittstelle, mittels derer beispielsweise eine Messung, umfassend die Emission von Abfragelicht und die Detektion von Antwortlicht, gestartet werden kann. Ebenfalls unter den Begriff der Initiierung zu umfassen sind Vorgänge, bei welchen beispielsweise permanent eine Emission von Abfragelicht oder eine Detektion von Antwortlicht erfolgt, wobei lediglich die jeweils andere dieser Funktionen durch das Lesegerät initiiert wird.

Das Lesegerät kann beispielsweise einen Hochfrequenz-Transmitter (RF-Transmitter) umfassen, beispielsweise einen Hochfrequenz-Transmitter, wie er üblicherweise in der RFID-Technologie eingesetzt wird. Dieser Hochfrequenz-Transmitter kann eingerichtet sein, um mit der oben beschriebenen, optionalen Hochfrequenzspule des Sensorpflasters zusammenzuwirken, beispielsweise indem die Frequenzen dieser Elemente aufeinander abgestimmt sind. Auf diese Weise kann ein unidirektionaler und/oder bidirektionaler Austausch von Daten und/oder Steuerbefehlen erfolgen. Der Hochfrequenz-Transmitter kann somit die Schnittstelle zwischen dem Lesegerät und dem Sensorpflaster darstellen und/oder einen Bestandteil dieser Schnittstelle bilden.

Das Sensorsystem kann auch auf komplexere Weise ausgestaltet sein. So kann beispielsweise das Sensorsystem eingerichtet sein, um mehrere Messungen zu verschiedenen Zeitpunkten durchzuführen, wobei punktuelle Messungen oder auch kontinuierliche Messungen umfasst sein können. Diese Ausführung von Messungen zu verschiedenen Zeitpunkten kann insbesondere auch automatisch erfolgen. Weiterhin kann das Sensorsystem eingerichtet sein, um aus den Messergebnissen dieser Messungen einen zeitlichen Konzentrationsverlauf einer Indikatorsubstanz in einem Körpergewebe und/oder einer Körperflüssigkeit zu ermitteln. Unter dem zeitlichen Konzentrationsverlauf kann dabei beispielsweise der vollständige oder abschnittsweise Verlauf der Konzentration verstanden werden, oder es können auch, alternativ oder zusätzlich, andere Größen bzw. Parameter ermittelt werden, welche den Konzentrationsverlauf charakterisieren. Als Beispiele derartiger Größen kann die Halbwertszeit genannt werden, wobei jedoch auch andere Größen alternativ oder zusätzlich einsetzbar sind. Derartige Größen werden im Folgenden allgemein als aus dem Konzentrationsverlauf abgeleitete Parameter bezeichnet.

Die Indikatorsubstanz kann dabei ausgestaltet sein wie in der obigen Beschreibung des Standes der Technik. Insbesondere kann die Indikatorsubstanz eine endogene und/oder eine exogene Indikatorsubstanz umfassen. Auf diese Weise kann mittels des vorgeschlagenen Sensorsystems beispielsweise eine Clearance der Indikatorsubstanz ermittelt werden, beispielsweise eine renale Clearance. Die Messergebnisse können dabei unmittelbar die Konzentrationen widerspiegeln, oder es kann sich bei den Messergebnissen um zu den Konzentrationen korrelierende Größen handeln, beispielsweise Fluoreszenzmessergebnisse, deren Intensitätswerte proportional zur Konzentration der Indikatorsubstanz in dem Körpergewebe und/oder der Körperflüssigkeit sein können. Auch andere Ausgestaltungen sind denkbar.

Zur Ermittlung des Konzentrationsverlaufs der Indikatorsubstanz können die Messergebnisse beispielsweise einfach gespeichert werden. Zu diesem Zweck können in dem Sensorpflaster und/oder dem Lesegerät beispielsweise ein oder mehrere flüchtige und/oder nicht-flüchtige Datenspeicher vorgesehen sein. Beispielsweise können die Messergebnisse als Messwertpaare in diesem Speicher gespeichert sein, beispielsweise in dem Lesegerät. So kann jedes Messwertpaar beispielsweise einen Zeitpunkt der Messung (beispielsweise angegeben in willkürlichen oder absoluten Zeiteinheiten) und einen oder mehrere zugehörige Messwerte des mindestens einen Detektors umfassen, beispielsweise eine gemessene Photospannung an einer Photodiode des Detektors. Diese Erfassung bedeutet somit, dass die Messergebnisse bzw. die Messwertpaare zusammengestellt und für eine spätere Abfrage bereitgestellt werden können. Alternativ oder zusätzlich kann jedoch auch bereits eine zumindest teilweise Aufbereitung der Messergebnisse in dem Sensorsystem erfolgen. So kann beispielsweise das Sensorsystem eingerichtet sein, um den Konzentrationsverlauf darzustellen, beispielsweise auf einem oder mehreren Displays des Sensorsystems, insbesondere des Lesegeräts. Ein Benutzer kann somit den Verlauf unmittelbar erkennen. Alternativ oder zusätzlich kann auch eine Analyse der Messergebnisse zumindest teilweise bereits in dem Sensorsystem erfolgen, so dass beispielsweise Eliminations-Halbwertszeiten, Clearance oder ähnliche Ergebnisse, welche sich aus dem Konzentrationsverlauf ermitteln lassen, bereits vollständig oder teilweise in dem Sensorsystem ermittelt werden können. Zu diesem Zweck kann das Sensorsystem beispielsweise in dem Sensorpflaster und/oder dem Lesegerät ein oder mehrere entsprechend eingerichtete Datenverarbeitungsgeräte umfassen. Das Sensorsystem kann auch mit einem oder mehreren weiteren Systemen zusammenwirken, beispielsweise einem oder mehreren externen Datenverarbeitungsgeräten. Zu diesem Zweck kann das Sensorsystem beispielsweise wiederum eine drahtgebundene und/oder drahtlose Schnittstelle aufweisen, mittels derer beispielsweise über einen Personal Computer, einen Server oder ähnliche Computersysteme die Messdaten bzw. Messergebnisse abgefragt werden können. Auf diese Weise kann eine weitergehende Auswertung in einem externen Computersystem erfolgen, oder beispielsweise ein behandelnder Arzt kann Zugriff auf die Messergebnisse haben.

Neben dem Sensorpflaster und dem Sensorsystem mit dem Sensorpflaster wird weiterhin ein Kit zur transkutanen Messung einer Organfunktion vorgeschlagen. Bei der Organfunktion kann es sich insbesondere wiederum um eine Nierenfunktion handeln. Das Kit umfasst mindestens ein Sensorpflaster gemäß einer oder mehreren der oben beschriebenen Formen. Alternativ oder zusätzlich kann das Kit auch ein komplettes Sensorsystem gemäß einer oder mehreren der oben beschriebenen Formen umfassen. Insofern kann für die möglichen Ausgestaltungen insgesamt auf die obige Beschreibung verwiesen werden. Die Sensorpflaster können beispielsweise einzeln oder zu mehreren verpackt sein, beispielsweise in einer Primärverpackung. Die übrigen Bestandteile des Kits können, beispielsweise gemeinsam mit einer Gebrauchsanleitung, in einer weiteren Verpackung, welche auch die Sensorpflaster umfassen kann, enthalten sein.

Weiterhin umfasst das Kit mindestens eine Indikatorsubstanz. Diese Indikatorsubstanz soll in dem Körper eines Patienten einbringbar sein, beispielsweise durch eine Injektion, eine orale Einnahme, eine transdermale Verabreichung oder eine rektale Verabreichung. Insofern soll die Indikatorsubstanz insbesondere die entsprechenden Verträglichkeiten mit dem Organismus eines menschlichen oder tierischen Patienten aufweisen, dessen Organfunktion gemessen werden soll.

Weiterhin soll die Indikatorsubstanz derart gewählt sein, dass deren zeitlicher Konzentrationsverlauf in dem Körper des menschlichen oder tierischen Patienten, insbesondere in einem Körpergewebe und/oder einer Körperflüssigkeit, als Indikator für die Organfunktion einsetzbar ist bzw. dienen kann. Beispielsweise kann es sich bei der Körperflüssigkeit um Blut, Urin oder vorzugsweise um interstitielle Flüssigkeit handeln.

Unter einer Indikatorsubstanz, deren Konzentrationsverlauf als Indikator für die Organfunktion einsetzbar ist, ist insbesondere eine Indikatorsubstanz zu verstehen, deren Konzentration zumindest weitgehend, vorzugsweise vollständig, lediglich von der zu beobachtenden Organfunktion abhängig ist. Wird beispielsweise eine Nierenfunktion, insbesondere eine glomeruläre Filtrationsrate, begutachtet, so wird als Indikatorsubstanz vorzugsweise ein beliebiger Stoff verwendet, welcher im Wesentlichen ausschließlich filtriert wird und weder in signifikanten Mengen tubulär sezerniert wird noch aus dem Primärharn rückresorbiert wird, noch im Körper metabolisiert wird.

Die Indikatorsubstanz soll dabei mindestens einen Marker umfassen, welcher eingerichtet ist, um bei Einstrahlung des mindestens einen Abfragelichts der Strahlenquelle des Sensorpflasters das mindestens eine Antwortlicht auszusenden. Wie oben dargestellt, können dabei mehrere Wirkmechanismen der Aussendung des Antwortlichts in Frage kommen. Insbesondere kann es sich bei diesen Mechanismen um eine Lumineszenz, insbesondere eine Fluoreszenz und/oder eine Phosphoreszenz, handeln. Auch andere Mechanismen sind jedoch grundsätzlich möglich, beispielsweise eine Lichtstreuung, beispielsweise eine Raman- und/oder Stokes-Streuung. Grundsätzlich sind auch andere Mechanismen möglich, beispielsweise eine Absorption und/oder eine Reflexion, vorzugsweise eine wellenlängenabhängige Absorption und/oder Reflexion. Insofern kann das Antwortlicht beispielsweise einen reflektierten, einen transmittierten oder einen gestreuten Lichtstrahl oder eine Kombination derartiger Lichtstrahlen umfassen. Alternativ oder zusätzlich kann das Antwortlicht auch ein Fluoreszenzlicht und/oder ein Phosphoreszenzlicht oder ein auf eine andere Weise bei der Wechselwirkung des Abfragelichts mit dem Marker entstehendes Antwortlicht umfassen.

Der Marker kann dabei ebenfalls auf unterschiedliche Arten ausgestaltet sein. So kann zum einen die Indikatorsubstanz als Ganzes als derartiger Marker ausgestaltet sein, so dass mittels des Abfragelichts beispielsweise spektroskopische Eigenschaften, also entsprechend eines oder mehreren der oben beschriebenen Wirkmechanismen der Wechselwirkung mit dem Abfragelicht, des gesamten Moleküls bzw. sämtlicher Moleküle der Indikatorsubstanz abgefragt werden können. Alternativ oder zusätzlich kann die Indikatorsubstanz jedoch auch lediglich den Marker als einen von mehreren Bestandteilen umfassen. So können beispielsweise ein oder mehrere Marker-Reste, Marker-Gruppen oder ähnlicher Markerbestandteile über eine Bindung an die Indikatorsubstanz gekoppelt sein. Beispielsweise kann es sich dabei um eine kovalente Bindung, eine Komplexbindung, eine ionische Bindung oder auch eine einfache Bindung über Van-der-Waals-Kräfte handeln. Der Marker kann beispielsweise ein fluoreszierendes Molekül umfassen, beispielsweise das oben beschriebene Fluorescein-Isothiocyanat (FITC).

Die offenbarte Indikatorsubstanz ist also vorzugsweise eine Fluoreszenz-markierte Indikatorsubstanz. Diese hat bevorzugt eine Struktur gemäß der allgemeinen Formel (I):

P-F (Formel I)

wobei P ein Polyol ist; und
wobei F ein Marker mit optisch messbaren Eigenschaften, insbesondere fluoreszierender und/oder phosphoreszierender Marker, ist.

Polyole für die Indikatorsubstanz umfassen vorzugsweise Polyethylenglykol, Ethylenglykol, Propylenglykol, Glycerin, Mannitol, Sorbitol, Hexite, Pentite, Tetrite, Inosite, Mannose, Aldosen, Laktose, Cellobiose, Gentiobiose, β-Alkylglykoside, Desoxyzucker, β-Alkyluronsäuren, Fucose, Desoxyzuckeralkohole, Fructose, sowie jeweils Derivaten wobei das Polyol als Desoxyaminozuckeralkohol vorliegt. Bevorzugt ist das Polyol ein Polysacharid, insbesondere bevorzugt Inulin oder Sinistrin und insbesondere ein Inulin oder Gemisch aus Inulinen, die von 3 bis 20, vorzugsweise 11 bis 15 oder 3 bis 8 Fructoseeinheiten aufweisen.

Vorzugsweise ist der Marker ausgewählt aus der Gruppe bestehend aus: Fluorescein-Farbstoffe, Cyanin-Farbstoffe, Naphthylamid-Farbstoffe, Coumarin-Farbstoffe, Xanthen-Farbstoffe, Thioxanthen-Farbstoffe, Naphtholacton-Farbstoffe, Azlacton-Farbstoffe, Methin-Farbstoffe, Oxazin-Farbstoffe, Thiazin-Farbstoffe. Bevorzugt ist F ein Fluorescein-Farbstoff, besonders bevorzugt Fluorescein.

Bevorzugt kann der fluoreszierende Marker über eine Kopplungsgruppe mit dem Polysacharid verbunden werden. Geeignete Kopplungsgruppen und Kopplungsreaktionen sind dem Fachmann bekannt. Besonders bevorzugt ist die Kopplungsgruppe ausgewählt aus der Gruppe bestehend aus: Thioharnstoff-Gruppe (-N-CS-N-), Thiocarbamat-Gruppe (-N-CS-O-), Carbamat-(Urethan-)-Gruppe (-N-CO-O-), Ether-Gruppe (-O-), Thioether-Gruppe (-S-), Ester-Gruppe (-CO-O-), Amid-Gruppe (-CO-N-), Thioester-Gruppe (-CS-O-), Thioamid-Gruppe (-CS-N-), Aminoalkyl-Gruppe (-CO-N-(CH₂)n-O-) mit n = 2 bis 5, sekundäre Amin-Gruppe (-NH-). Insbesondere liegt der fluoreszierende Marker als Fluorescinisothiocyanat (FITC) vor.

Derartige Substanzen lassen sich insbesondere für die Nierenfunktionsmessung einsetzen, wie auch andere Indikatorsubstanzen, welche ausschließlich über den Harnweg im menschlichen Körper eliminiert werden. Besonders bevorzugt ist die Verwendung Fluoreszenz-markierter Polysaccharide und/oder Cyclosaccharide, wie beispielsweise Sinistrinen und/oder Fructosanen, welche beispielsweise mit FITC markiert sind. Für die Herstellung derartiger markierter Polysaccharide und/oder Cyclosaccharide kann beispielsweise auf den obigen Stand der Technik verwiesen werden, beispielsweise die WO2001/85799 oder WO2006/32441.

Neben dem Sensorpflaster, dem Sensorsystem oder dem Kit, jeweils in einer oder mehreren der oben beschriebenen Formen, wird weiterhin die Verwendung eines oder mehrerer dieser Vorrichtungen für eine transkutane Messung einer Organfunktion vorgeschlagen. Insbesondere kann es sich dabei um eine Nierenfunktion, insbesondere eine glomeruläre Filtrationsrate, handeln.

Entsprechend wird ein Verfahren zur transkutanen Messung einer Organfunktion vorgeschlagen, insbesondere einer Nierenfunktion. Dieses Verfahren kann insbesondere unter Verwendung eines Sensorpflasters und/oder eines Sensorsystems und/oder eines Kits gemäß einer oder mehrerer der oben beschriebenen Formen durchgeführt werden, so dass für mögliche Ausgestaltungen des Verfahrens weitgehend auf die obige Beschreibung verwiesen werden kann.

Das Verfahren umfasst die folgenden Schritte, welche vorzugsweise, jedoch nicht notwendigerweise, in der im Folgenden dargestellten Reihenfolge durchgeführt werden. Es können auch zusätzliche, nicht dargestellte Verfahrensschritte durchgeführt werden und/oder es können einzelne oder mehrere der Verfahrensschritte zeitlich parallel, zeitlich überlappend oder auch wiederholt durchgeführt werden.

In einem ersten Verfahrensschritt wird ein Sensorpflaster auf eine Körperoberfläche aufgebracht, insbesondere aufgeklebt. Das Sensorpflaster umfasst mindestens eine Strahlenquelle, vorzugsweise als integralen Bestandteil, wobei die Strahlenquelle eingerichtet ist, um die Körperoberfläche mit mindestens einem Abfragelicht zu bestrahlen. Das Sensorpflaster umfasst weiterhin einen Detektor, vorzugsweise ebenfalls als integralen Bestandteil, welcher eingerichtet ist, um mindestens ein aus Richtung der Körperoberfläche eingestrahltes Antwortlicht zu erfassen.

In einem weiteren Verfahrensschritt werden mindestens zwei zeitlich begrenzte Messungen zu verschiedenen Zeitpunkten und/oder mindestens eine kontinuierliche Messung über einen Zeitraum hinweg durchgeführt, wobei das Antwortlicht zu den verschiedenen Zeitpunkten bzw. über den Zeitraum hinweg erfasst wird. Insofern lassen sich beispielsweise wiederum, wie oben beschrieben, Messwertpaare bilden, bei welchem einem Zeitpunkt ein oder mehrere Messwerte des Detektors zugeordnet werden, beispielsweise entsprechende Sensorsignale. Die Erfassung kann ebenfalls gemäß der obigen Beschreibung erfolgen, so dass beispielsweise eine Speicherung und/oder Bereitstellung dieser Messwertpaare erfolgen kann.

In einem dritten Verfahrensschritt wird dann aus einem zeitlichen Verlauf des Antwortlichts auf einen zeitlichen Verlauf einer Konzentration einer Indikatorsubstanz geschlossen. Dabei kann der zeitliche Verlauf des Antwortlichts kontinuierlich oder punktweise bekannt sein. So kann, wie oben dargestellt, der zeitliche Verlauf beispielsweise kontinuierlich gemessen werden. Alternativ oder zusätzlich kann jedoch auch eine Extrapolation und/oder Interpolation einzelner Messwerte erfolgen, beispielsweise durch Anpassung einer oder mehrerer der Messkurven. Beispielsweise kann diese Anpassung bereits vollständig oder teilweise im Sensorpflaster erfolgen und/oder in einem Lesegerät des Sensorsystems. Auch andere Ausgestaltungen sind denkbar, beispielsweise eine nachträgliche externe Auswertung in einem separaten Computersystem.

Wie oben dargestellt, soll das Verfahren insbesondere derart vorgenommen werden, dass das Antwortlicht zur Konzentration der Indikatorsubstanz korreliert. Dabei können beispielsweise die oben dargestellten Wechselwirkungsmechanismen zwischen dem Abfragelicht und der Indikatorsubstanz und/oder einem Marker der Indikatorsubstanz ausgenutzt werden, beispielsweise ein Fluoreszenzmechanismus. Da, beispielsweise aus Kalibrationsmessungen und/oder empirischen oder semi-empirischen oder theoretischen Überlegungen, ein Zusammenhang zwischen der Konzentration der Indikatorsubstanz und dem Antwortlicht, beispielsweise eine Intensität des Antwortlichts und/oder einem Detektorsignal des Detektors, bekannt ist oder ermittelt werden kann, ist dieser Rückschluss aus dem zeitlichen Verlauf des Antwortlichts auf die Konzentration der Indikatorsubstanz für den Fachmann leicht zu bewerkstelligen. Beispielsweise kann dieses Umrechnen in die Konzentration der Indikatorsubstanz in willkürlichen Einheiten erfolgen, so dass beispielsweise die Intensität des Antwortlichts unmittelbar als Maß für die Indikatorsubstanz verwendet werden kann. Alternativ oder zusätzlich kann jedoch auch eine andere Art von Umrechnung erfolgen, beispielsweise mittels einer oder mehrerer hinterlegter Umrechnungskurven, Umrechnungsalgorithmen oder Umrechnungstabellen, welche beispielsweise in einem oder mehreren Datenverarbeitungsgeräten eingesetzt werden können. So kann beispielsweise diese Umrechnung vollständig oder teilweise in einem Datenverarbeitungsgerät des Sensorpflasters und/oder in einem Datenverarbeitungsgerät des Lesegeräts und/oder in einem weiteren, externen Datenverarbeitungsgerät erfolgen.

Wie oben dargestellt, kann es sich bei der Indikatorsubstanz um eine endogene oder exogene Indikatorsubstanz handeln. Insofern kann diese Indikatorsubstanz beispielsweise ohnehin im Körper des menschlichen oder tierischen Patienten vorhanden sein und/oder kann durch künstliche Aufnahme der Indikatorsubstanz, beispielsweise durch orale Einnahme, durch rektale Verabreichung oder durch Injektion, kurzfristig in ihrer Konzentration künstlich erhöht werden, um dann die Zufuhr abzubrechen. Alternativ oder zusätzlich kann auch beispielsweise eine Zufuhr der Indikatorsubstanz derart geregelt werden, dass der zeitliche Verlauf der Konzentration der Indikatorsubstanz im Wesentlichen konstant ist, wobei aus der erforderlichen Nachfuhrrate, beispielsweise gemessen in Mengeneinheiten oder Masseneinheiten pro Zeiteinheit, auf die entsprechende Organfunktion geschlossen werden kann. Auch die soll von dem Gedanken umfasst sein, dass aus dem zeitlichen Verlauf des Antwortlichts auf den zeitlichen Verlauf der Konzentration der Indikatorsubstanz geschlossen wird. Verschiedene andere Messmethoden sind denkbar. Entsprechend kann die Zufuhr der Indikatorsubstanz Bestandteil des vorgeschlagenen Verfahrens sein.

Insgesamt weisen die vorgeschlagenen Vorrichtungen und Verfahren gegenüber bekannten Vorrichtungen oder Verfahren dieser Art eine Vielzahl von Vorteilen auf, welche einzeln oder in Kombination realisierbar sind. So kann beispielsweise das Sensorpflaster als druckbares, intelligentes Sensor-Heftpflaster auf Elektronik-Basis ausgestaltet sein. Es lässt sich somit ein Sensorpflaster mit geringen Herstellungskosten realisieren, da beispielsweise Druckverfahren mit Großdruckmaschinen einsetzbar sind. Dabei lassen sich auch preiswerte Rohmaterialien einsetzen, wie beispielsweise kostengünstige organische Polymere für den Detektor und/oder die Strahlenquelle bzw. Lichtquelle und/oder sonstige Bestandteile der Elektronik, beispielsweise der Auswerteelektronik.

Weiterhin lassen sich für den Detektor, die Datenverarbeitung, die Speicherung und die Schnittstelle oder Kombinationen dieser und/oder anderer Elemente Standardelemente einsetzen, welche auch in anderen Konfigurationen wieder verwendet werden können. Insofern lässt sich ein Baukastensystem realisieren, was ebenfalls wiederum zu verringerten Herstellungskosten, verringerten Lagerhaltungskosten und somit insgesamt zu einer Kostensenkung führen kann.

Das Sensorpflaster lässt sich somit insbesondere als hochintegriertes Heftpflaster ausgestalten. Die Abmessungen dieses Heftpflasters können den Abmessungen üblicher Heftpflaster entsprechen, also beispielsweise im Bereich zwischen 5 bis 100 mm x 5 bis 100 mm liegen. Das Sensorpflaster kann sich aus einer optischen Einheit in Form der Strahlenquelle zusammensetzen, beispielsweise einer Leuchtdiode, einem Laser oder Ähnlichem, sowie einem oder mehreren Detektoren, welche ebenfalls der optischen Einheit zugeordnet sein können. Dieser Detektor kann, wie oben dargestellt, beispielsweise eine Photodiode und/oder eine Solarzelle umfassen. Die optische Einheit mit der Strahlenquelle und dem Detektor kann beispielsweise als eigenständige Einheit ausgebildet sein, welche beispielsweise auch räumlich zusammenhängend auf dem Sensorpflaster aufgebracht sein kann. Diese optische Einheit lässt sich beispielsweise mit einer oder mehreren Filterfolien und/oder mit durch Presstechnik bzw. Drucktechniken erzeugten optischen Abbildungssystemen, beispielsweise Fresnel-Linsen, kombinieren. Auf diese Weise lässt sich eine zuverlässig arbeitende, kostengünstige und äußerst kleinvolumige optische Einheit herstellen, welche einen hohen Integrationsgrad aufweist.

Neben der optischen Einheit kann eine elektronische Einheit vorgesehen sein, welche beispielsweise die oben beschriebene Auswerteelektronik umfassen kann. Diese kann beispielsweise geeignete Verstärker, Wandler (beispielsweise A/D-Wandler), Controller, Speicherelemente oder Kombinationen der genannten und/oder anderer Bauelemente umfassen.

Alternativ oder zusätzlich zu der elektronischen Einheit und neben der optischen Einheit kann das Sensorpflaster weiterhin eine oder mehrere Kommunikationseinheiten umfassen. Beispielsweise kann es sich dabei, wie oben dargestellt, um eine Kommunikationseinheit auf RFID-Basis handeln. Diese kann beispielsweise eine oder mehrere Hochfrequenzspulen umfassen. Die Kommunikationseinheit kann mit der optionalen elektronischen Einheit und/oder der optischen Einheit funktional zusammenwirken.

Neben der optischen Einheit und der optionalen elektronischen Einheit und/oder der optionalen Kommunikationseinheit kann das Sensorpflaster weitere Elemente umfassen, wie beispielsweise den elektrischen Energiespeicher und/oder die Energieerzeugungsvorrichtung, wie beispielsweise die Solarzelle. Auch andere Elemente können umfasst sein, beispielsweise Anzeigeelemente oder Ähnliches, was es einem Benutzer ermöglicht, Informationen und/oder Kontrollsignale mit dem Sensorpflaster auszutauschen.

Der Aufbau des offenbarten Sensorpflasters kann vergleichsweise einfach erfolgen. So kann beispielsweise neben der mindestens einen Haftoberfläche, beispielsweise mit zwei Klebebereichen, im Zentrum des Sensorpflasters jeweils mindestens ein Detektor, beispielsweise mindestens eine Solarzelle, und mindestens eine Strahlenquelle, beispielsweise mindestens eine OLED, aufgedruckt werden. Vor diesen optischen Elementen können sich jeweils geeignete Filterfolien befinden, welche beispielsweise verhindern können, dass Abfragelicht von dem Detektor in erheblichem Maße mitdetektiert wird. Neben und/oder hinter der optischen Einheit mit dem Detektor und der Strahlenquelle kann sich die Ansteuerelektronik für diese optische Einheit befinden. Diese Ansteuerelektronik kann, wie oben ausgeführt, ebenfalls wiederum als kostengünstige gedruckte Ansteuerelektronik ausgestaltet sein und kann eine Ansteuerung für den Detektor und/oder die Strahlenquelle enthalten. Auch eine Vorrichtung zur Digitalisierung der Messsignale, beispielsweise der von dem Detektor erzeugten Signale, kann vorgesehen sein. Weiterhin können, alternativ oder zusätzlich, auch ein oder mehrere Speicherelemente und/oder eine Steuerelektronik für das Auslesen, beispielsweise über Hochfrequenzsignale, vorgesehen sein.

Ebenfalls in Schichttechnologie kann die Schnittstelle, beispielsweise mit der Hochfrequenzspule, erzeugt werden. Diese kann beispielsweise wiederum in einer darüber liegenden Schichtebene erzeugt werden und kann Hochfrequenzsignale erzeugen, welche dann ausgelesen werden können. Beispielsweise kann das Lesegerät ein konventionelles RFID-Lesegerät zum Auslesen der Hochfrequenzsignale umfassen. Von dem Lesegerät kann dann eine Übertragung dieser Informationen erfolgen, beispielsweise in eine geeignete Datenbank, welche Bestandteil des Lesegeräts oder Bestandteil eines separaten Geräts sein kann. Von dieser Datenbank aus kann dann später beispielsweise eine weitere Auswertung der Messsignale bzw. Messergebnisse erfolgen.

Die für die Aufnahme der Messsignale erforderliche Energie kann ganz oder teilweise von dem optionalen Energiespeicher bereitgestellt werden, welcher beispielsweise ebenfalls in das Sensorpflaster integriert sein kann. Beispielsweise kann dieser elektrische Energiespeicher wiederum vollständig oder teilweise in Polymer-Technik aufgebaut sein, beispielsweise vollständig oder teilweise als Polymer-Batterie. Für das Aufbringen dieser Polymer-Batterie kann beispielsweise wiederum eine Drucktechnik verwendet werden. Alternativ oder zusätzlich sind auch andere Arten von Energiespeichern einsetzbar, beispielsweise konventionelle Dünnfilm-Energiespeicher. Wiederum alternativ oder zusätzlich können jedoch auch andere Energiequellen genutzt werden, beispielsweise Energiequellen, welche extern gelagert sind und welche über eine oder mehrere Schnittstellen mit dem Sensorpflaster verbunden sein können. So kann beispielsweise eine drahtlose Energieübertragung an das Sensorpflaster erfolgen und/oder eine Übertragung mittels eines (beispielsweise an das Sensorpflaster ansteckbaren) Energieversorgungskabels.

Das Trägermaterial bzw. das mindestens eine Trägerelement des Sensorpflasters kann, neben der Bereitstellung der mindestens einen Haftfläche zum Aufkleben auf die Körperoberfläche des menschlichen oder tierischen Patienten, weitere Aufgaben übernehmen. So kann das Trägermaterial beispielsweise derart gewählt werden, dass dieses im Wesentlichen lichtdichte Eigenschaften aufweist, so dass beispielsweise kein störendes Streulicht, beispielsweise Umgebungslicht, durch das Trägermaterial zu dem Detektor und/oder zu der mit dem Abfragelicht zu bestrahlenden Körperoberfläche gelangen kann. Auf diese Weise lässt sich ein störender Streulichthintergrund unterdrücken. Weiterhin können das Sensorpflaster, beispielsweise das Trägerelement und beispielsweise dessen Haftflächen, derart ausgestaltet sein, dass seitlich kein Licht, beispielsweise Umgebungslicht, eindringen kann. Beispielsweise kann dies dadurch erfolgen, dass die Haftflächen die optische Einheit, also den Detektor und/oder die Strahlenquelle, vollständig in der Ebene der Körperoberfläche umschließen. Auf diese Weise kann ebenfalls ein Eindringen von Streulicht und/oder Umgebungslicht verhindert werden. Weiterhin können auch der verwendete Klebstoff und/oder sonstige Materialien des Sensorpflasters lichtdicht ausgestaltet sein, also derart, dass diese weitgehend intransparent oder mit geringer Transparenz ausgestaltet sind für Licht im spektralen Bereich des Abfragelichts und/oder des Antwortlichts.

Das Sensorsystem kann beispielsweise in Betrieb genommen werden, indem ein Hochfrequenzpuls, beispielsweise ausgesandt von dem Lesegerät, das Sensorpflaster, beispielsweise eine Ansteuerelektronik des Sensorpflasters, initiiert bzw. aktiviert. Hierdurch kann das Sensorpflaster zur Aufnahme von Messdaten angeregt werden. Diese Messdaten können beispielsweise digitalisiert werden und in einen oder mehrere Speicherelemente eingetragen werden. Wie oben beschrieben, können diese ein oder mehrere Speicherelemente als Messwertspeicher, beispielsweise als flüchtiger und/oder nichtflüchtiger Speicher ausgestaltet sein, beispielsweise als Speicher vom Flash-Typ. Dieses mindestens eine Speicherelement kann beispielsweise ebenfalls in dem Sensorpflaster enthalten sein. In letzterem Fall können diese Daten dann beispielsweise von dem Lesegerät, beispielsweise wiederum mittels Hochfrequenztechnik, ausgelesen werden. Alternativ oder zusätzlich kann auch eine zumindest teilweise Datenverarbeitung bereits auf dem Sensorpflaster erfolgen, so dass bereits zumindest teilweise verarbeitete Daten an das Lesegerät weitergegeben werden können. Wiederum alternativ können auch vollständig rohe Daten, beispielsweise unmittelbar von dem Detektor generierte Daten, bereits an das Lesegerät weitergegeben werden, um dort in einem oder mehreren Speicherelementen gespeichert zu werden. Verschiedene Kombinationen sind denkbar.

Bei der Ausgestaltung des Detektors und/oder der Strahlenquelle und/oder der Auswerteelektronik oder sonstiger elektronischer Komponenten des Sensorpflasters können, wie oben bereits teilweise erwähnt, die Komponenten einzeln oder in Gruppen derart ausgelegt werden, dass wiederholbare Messsituationen möglich sind. So ist es insbesondere, wie oben dargestellt, bevorzugt, wenn kalibrierte Strahlenquellen, beispielsweise kalibrierte Leuchtdioden und/oder Laser, eingesetzt werden. Alternativ oder zusätzlich können auch entsprechend kalibrierte Detektoren eingesetzt werden. Weiterhin lassen sich, ebenfalls alternativ oder zusätzlich, auch weitere elektronische Komponenten als kalibrierte Komponenten ausgestalten. Beispielsweise lassen sich kalibrierte Verstärker und/oder A/D-Wandler einsetzen. Um die Messsituation weiter wiederholbar zu gestalten, können auch fehlertolerante und/oder redundante elektrische Schaltkreise eingesetzt werden, welche beispielsweise auch kompensatorisch ausgestaltet sein können. Auf diese Weise kann die Funktionsfähigkeit sichergestellt werden. Weiterhin lassen sich auch Testschaltkreise einsetzen, um während der Kalibration die notwendigen Parameter intern zu speichern und das System sich entsprechend selbst konfigurieren zu lassen. Auf diese Weise lassen sich beispielsweise defekte Elemente umgehen, lassen sich Lastwiderstände einstellen oder Ähnliches. Die Auswerteschaltung kann optional ein oder mehrere derartiger Testschaltkreise umfassen. Insgesamt lassen sich somit die vorgeschlagenen Vorrichtungen störungsunempfindlich ausgestaltet und ermöglichen sichere und reproduzierbare Messungen.

Grundsätzlich betrifft die vorliegende Erfindung auch die Verwendung einer Fluoreszenz-markierten Indikatorsubstanz zur Herstellung eines Diagnostikums zur Bestimmung der glomerulären Filtrationsrate (GFR) gemäß einem der Ansprüche 1 bis 3.

Die erfindungsgemäß in diesem Zusammenhang verwendete Fluoreszenz-markierte Indikatorsubstanz ist ein Gemisch aus Inulinen, die 11 bis 15 oder 3 bis 8 Fructoseeinheiten aufweisen, wobei die Inuline mit einem fluoreszierenden Marker gekoppelt sind. Der fluoreszierende Marker ist vorzugsweise ausgewählt aus der Gruppe bestehend aus: Fluorescein-Farbstoffe, Cyanin-Farbstoffe, Naphthylamid-Farbstoffe, Coumarin-Farbstoffe, Xanthen-Farbstoffe, Thioxanthen-Farbstoffe, Naphtholacton-Farbstoffe, Azlacton-Farbstoffe, Methin-Farbstoffe, Oxazin-Farbstoffe, Thiazin-Farbstoffe. Bevorzugt ist F ein Fluorescein-Farbstoff, besonders bevorzugt Fluorescein.

Bevorzugt kann der fluoreszierende Marker über eine Kopplungsgruppe mit dem Polysaccharid verbunden werden. Geeignete Kopplungsgruppen und Kopplungsreaktionen sind dem Fachmann bekannt. Besonders bevorzugt ist die Kopplungsgruppe ausgewählt aus der Gruppe bestehend aus: Thioharnstoff-Gruppe (-N-CS-N-), Thiocarbamat-Gruppe (-N-CS-O-), Carbamat-(Urethan-)-Gruppe (-N-CO-O-), Ether-Gruppe (-O-), Thioether-Gruppe (-S-), Ester-Gruppe (-CO-O-), Amid-Gruppe (-CO-N-), Thioester-Gruppe (-CS-O-), Thioamid-Gruppe (-CS-N-), Aminoalkyl-Gruppe (-CO-N-(CH₂)n-O-) mit n = 2 bis 5, sekundäre Amin-Gruppe (-NH-). Insbesondere liegt der fluoreszierende Marker als Fluorescinisothiocyanat (FITC) vor.

Bevorzugt kann das Inulin-Gemisch durch enzymatischen Verdau und anschließende chromatographische Auftrennung von natürlich vorkommendem Inulin gewonnen werden. Durch den enzymatischen Verdau mit einer ß-Glucosidase, vorzugsweise Inulinase [E.C.: 3.2.1.7] und die anschließende Chromatographie können gezielt Gemische von Inulin mit einem Polymerisierungsgrad (d.h. Anzahl an Sacharidmonomereinheiten im Polysacharid) zwischen 3 und 20 und vorzugsweise zwischen 3 und 8 oder 11 und 15 bereitgestellt werden. Je nach Beschaffenheit des Ausgangsmaterials, können entsprechende Inulin-Gemische auch nur durch chromatographische Auftrennung gewonnen werden.

Die Fluoreszenz-markierte Indikatorsubstanz wird erfindungsgemäß als Diagnostikum formuliert. Hierbei wird eine definierte Menge, die ausreichend ist, um ein nachweisbares Fluoreszenz-Signal nach Verabreichung zu erzeugen, in einem physiologisch verträglichen Lösungsmittel, z.B. Wasser oder wässrige Salzlösungen, PBS etc., gelöst und ggf. mit physiologisch verträglichen Hilfsstoffen, z.B. Stabilisatoren, versetzt. Es versteht sich, dass die Menge an Fluoreszenz-markierter Indikatorsubstanz je nach Verwendung des Diagnostikums und je nach zu untersuchendem Probanden unterschiedlich sein kann. Faktoren, die in diesem Zusammenhang eine Rolle spielen können, sind Körpergewicht, Alter, Geschlecht, Art und Ausmaß der Nierenfunktionsstörung oder vermuteten Nierenfunktionsstörung, und/oder Krankengeschichte. Ein Diagnostikum im Sinne der vorliegenden Erfindung kann schließlich noch Angaben über die Art, Dauer, Umfang und Nebenwirkungen der Verwendung enthalten, die in Form eines Beipackzettels oder in elektronischer Form, z.B. auf einem Datenträger, beigefügt sein können. Ferner kann der Beipackzettel oder der Datenträger Angaben enthalten, die eine Interpretation der GFR erlauben.

Der Begriff der glomerulären Filtrationsrate (GFR) wurde bereits anderswo in der Beschreibung ausführlich definiert. Die Bestimmung der GFR dient erfindungsgemäß vorzugsweise dazu, bestehende Nierenfunktionsstörungen zu diagnostizieren, das Risiko für zukünftige Progression der Nierenfunktionsstörungen zu bestimmen, zum Überwachen ("Monitoring") bei Erkrankungen, therapeutischen Eingriffen oder Therapien, die Nierenfunktionsstörungen verursachen können, oder zur Bestimmung der individuellen Dosis für Arzneimittel, die über die Niere ausgeschieden werden. Unter Nierenfunktionsstörungen sind alle pathologischen Veränderungen der Nierenfunktion zu verstehen, die eine geänderte und vorzugsweise erniedrigte, aber auch erhöhte GFR zur Folge haben. Hierzu zählen vorzugsweise chronische Nierenfunktionsstörungen sowie akutes Nierenversagen, aber auch eine Hyperfiltration z.B. bei schlecht eingestelltem Diabetes mellitus. Nierenfunktionsstörungen können aber auch als Sekundärstörungen durch andere Erkrankungen hervorgerufen werden. So können Nierenfunktionsstörungen auch bei Vorliegen von kardiovaskulären Erkrankungen oder einer Prädisposition für das Auftreten von kardiovaskulären Erkrankungen und bei Diabetes mellitus order renalis auftreten.

Je nach Zweck der Bestimmung der GFR kann das Diagnostikum als Bolus oder durch Infusion verabreicht werden. Entsprechend können verschiedene Aspekte der GFR gemessen werden wie die sogenannte Input-Clearance, Infusions-Clearance oder Bolus-Clearance.

Vorteilhafterweise eignen sich die hier offenbarten Diagnostika zur nicht-invasiven, transkutanen Messung der GFR. Die Fluoreszenz-markierten Indikatorsubstanzen dringen nach Verabreichung in den interstitiellen Raum, wo eine störungsfreie Bestimmung der Fluoreszenz nach Anregung möglich ist. Die Bestimmung erfolgt vorzugsweise mit einer Vorrichtung wie anderswo in der Beschreibung offenbart, kann aber auch mit anderen im Stand der Technik bekannten Verfahren und Vorrichtungen zur Quantifizierung von fluoreszierenden Substanzen erfolgen. Ein weiterer Vorteil der erfindungsgemäß verwendeten Diagnostika ist, dass die Fluoreszenz-markierte Indikatorsubstanz aus einem definierten Gemisch von Inulinen, besteht. Dies erlaubt eine Standardisierung der GFR Bestimmung, die bisher problematisch war, da Inulin zwar Goldstandard zur Bestimmung der GFR ist, jedoch aufgrund wechselnder Zusammensetzung Nachteile bezüglich der Standardisierung hat. Durch den Einsatz kleinerer Polymere kann zudem die Löslichkeit insbesondere in Wasser und wässrigen Lösungen erhöht werden. Ausfällungsprobleme, die auch in der Folge zu klinischen Nebenwirkungen führen, können ebenfalls vermieden werden. Durch die erhöhte Löslichkeit können zudem kleinere Volumina als Diagnostikum verabreicht werden, was die Bioverträglichkeit zusätzlich erhöht. Durch die Verwendung kleinerer Polymere wird außerdem noch ein optimaler Grad an Markierung mit dem Fluoreszenz-Marker bezogen auf das Gesamtmolekül erreicht, was erlaubt, die Menge an Fluoreszenz-markierter Indikatorsubstanz im Diagostikum zu reduzieren. Es muss also weniger Indikatorsubstanz verabreicht werden, da die Fluoreszenzmarkierung im gleichen Volumen häufiger vorkommt. Schließlich wird durch das Verhältnis von Marker zu Polymer in den erfindungsgemäß als Diagnostikum zu verwendenden Fluoreszenz-markierter Indikatorsubstanzen auch deren lipophile Eigenschaften erhöht. Dadurch wird die renale Exkretionsrate erniedrigt und die Halbwertszeit im Organismus erhöht.

Die Offenbarung betrifft schließlich auch ein Verfahren zur Bestimmung der glomerulären Filtrationsrate (GFR), das die folgenden Schritte umfasst:
a. Verabreichen einer Fluoreszenz-markierten Indikatorsubstanz, vorzugsweise eines Gemisches von Inulinen, wie oben ausgeführt, an einen Probanden;
b. Messung der Fluoreszenz nicht-invasiv auf der Körperoberfläche; und
c. Bestimmung der GFR basierend auf den Messwerten aus Schritt b.

Das offenbarte Verfahren wird vorzugsweise nicht-invasiv durchgeführt. Hierzu kann die offenbarte Vorrichtung eingesetzt werden. Es können jedoch auch andere im Stand der Technik bekannte Systeme zur Fluoreszenz Messung verwendet werden. Wie bereits ausgeführt wurde, kann die GFR - je nach weiterem Verwendungszweck - als Input-Clearance, Infusions-Clearance oder Bolus-Clearance bestimmt werden. Dementsprechend kann die Verabreichung als Bolusgabe, als Infusion oder als Mischform erfolgen. Auch kann die Messung eine einmalig Messung (Bestimmung der Fluoreszenz zu einem bestimmten Zeitpunkt) oder eine mehrmalige Messung sein (Bestimmung der Fluoreszenz zu mehreren Zeitpunkten zur Verlaufsdarstellung).

Die GFR kann relativ oder absolut bestimmt werden. Unter relativer Bestimmung ist im Sinne der vorliegenden Erfindung die Bestimmung einer Änderung, d.h. einer Zunahme oder Abnahme der GFR zu verstehen. Diese kann ggf. auch als prozentuale Änderung von einem Ausgangswert ausgedrückt werden. Die Bestimmung der absoluten GFR setzt voraus, dass zunächst eine Kalibrierung für die Indikatorsubstanz durchgeführt wird, die es erlaubt, einem bestimmten gemessenen Fluoreszenzwert eine bestimmte Konzentration an Indikatorsubstanz im Blut, Plasma oder Serum zuzuordnen. Basierend auf dieser Konzentration, kann dann die GFR mit den im Stand der Technik bekannten Formeln berechnet werden.

Das Verfahren kann teilweise automatisiert werden. Zur Messung können, wie bereits erwähnt, die Vorrichtungen der vorliegenden Offenbarung eingesetzt werden. Die Auswertung und Berechnung der GFR kann Computer-gestützt erfolgen.

In einer weiteren Form dieses Verfahrens kann auch eine Diagnosestellung basierend auf der GFR erfolgen. Eine statistisch signifikant erniedrigte GFR ist vorzugsweise ein Indikator für eine Nierenfunktionsstörung oder eine Prädisposition dafür. Auch kann eine statistisch signifikante Erniedrigung der GFR ein Indikator dafür sein, die Dosierung von Arzneimitteln, die über die Niere ausgeschieden werden, zu senken. Umgekehrt kann eine erhöhte GFR ein Indikator dafür sein, dass keine Nierenfunktionsstörung oder Prädisposition dafür vorliegt. Die erhöhte GFR zeigt auch die Notwendigkeit an, die Dosierung von Arzneimitteln, die über die Niere ausgeschieden werden, zu erhöhen. Solche diagnostischen Auswertungen der durch das offenbarte Verfahren bestimmten GFR können natürlich auch automatisiert, z.B. durch den Einsatz eines auf einem Computer implementierten diagnostischen Algorithmus, erfolgen.

Das offenbarte Sensorpflaster oder Sensorsystem und der erfindungsgemäße Kit gemäß einem der Ansprüche 4 bis 13 kann auch zur transkutanen Messung einer Organfunktion verwendet werden, die eine funktionierende Barriere zwischen Blutgefäßsystem und extra-vasalen Räumen voraussetzen. Bevorzugt können Sensorpflasters, Sensorsystems oder Kit zur transkutanen Messung der Darmwandbarrierenfunktion oder der Blut-Hirn-Schrankenfunktion verwendet werden. Die Barrierefunktion kann hierbei durch Bestimmung der Zu oder Abnahme von Fluoreszenz-markierter Indikatorsubstanz im Blut bestimmt werden. Es versteht sich dabei, dass es bei gestörter Barrierefunktion zu einer verstärkten Abnahme der Fluoreszenz-markierter Indikatorsubstanz im Blut kommen wird. Umgekehrt ist nach oraler Verabreichung der Fluoreszenz-markierten Indikatorsubstanz bei vorliegender Barrierestörung ein Anstieg der Fluoreszenz im Blut möglich.

Die Offenbarung betrifft somit auch ein Verfahren zur transkutanen Messung der Darmwandbarrierenfunktion oder der Blut-Hirn-Schrankenfunktion, insbesondere unter Verwendung eines Sensorpflasters (116) nach einem der vorhergehenden, ein Sensorpflaster (116) betreffenden Offenbarungen und/oder eines Sensorsystems (114) nach einem der vorhergehenden, ein Sensorsystem (114) betreffenden Offenbarungen und/oder eines Kits (110) gemäß einem der Ansprüche 4 bis 13 wobei das Verfahren folgende Schritte umfasst:
- ein Sensorpflaster (116) wird auf eine Körperoberfläche aufgebracht, insbesondere aufgeklebt, wobei das Sensorpflaster (116) mindestens eine Strahlenquelle umfasst, wobei die Strahlenquelle eingerichtet ist, um die Körperoberfläche mit mindestens einem Abfragelichts (162) zu bestrahlen, wobei das Sensorpflaster (116) weiterhin einen Detektor (146) umfasst, wobei der Detektor (146) eingerichtet ist, um mindestens ein aus Richtung der Körperoberfläche eingestrahltes Antwortlicht (176) zu erfassen;
- mindestens zwei zeitlich begrenzte Messungen zu verschiedenen Zeitpunkten und/oder mindestens eine kontinuierliche Messung über einen Zeitraum hinweg werden durchgeführt, wobei das Antwortlicht (176) zu den verschiedenen Zeitpunkten und/oder über den Zeitraum hinweg erfasst wird; und
- aus einem zeitlichen Verlauf des Antwortlichts (176) wird auf einen zeitlichen Verlauf einer Konzentration einer Indikatorsubstanz (112) geschlossen.

Vorzugsweise wird bei den zuvor genannten Verfahren oder Verwendungen das offenbarte Gemisch aus Inulinen oder ein FITC Inulin oder ein FITC Sinistrin eingesetzt.

Die Offenbarung betrifft aber auch die Verwendung einer Fluoreszenz-markierten Indikatorsubstanz und vorzugsweise des erfindungsgemäßen Gemisch aus Inulinen oder eines FITC Inulin oder eines FITC Sinistrin zur Herstellung eines Diagnostikums zur Diagnose von Funktionsstörungen der Darmwandbarriere oder der Blut-Hirn-Schranke.

Das Auftreten von Funktionsstörungen der Darmwandbarriere steht hierbei vorzugsweise in Zusammenhang mit dem Auftreten Morbus Crohn oder Colitis ulcerosa, so dass die zuvor genannten Verwendungen und Verfahren grundsätzlich auf zur Diagnose dieser Erkrankungen eingesetzt werden können.

Funktionsstörungen der Blut-Hirn-Schranke treten in Zusammenhang mit verschiedenen Erbkrankheiten auf können jedoch auch in Zusammenhang mit anderen Erkrankungen stehen, z.B. neurodegenerativen Erkrankungen, Enzündungen des ZNS oder Schlaganfall. Als Erbkrankheiten mit Störungen der Barrierefunktion der Blut-Hirn-Schranke kommen bevorzugt in Betracht das GLUT1 Defizit Syndrom, die hereditäte Folat-Malabsorption oder die Biotin-ansprechende Basalganglienerkrankung.

Sensorpflasters, Sensorsystems oder der Kit gemäß einem der Ansprüche 4 bis 13 können auch zur Bestimmung der Pankreasfunktion eingesetzt werden. Hierbei wird die Funktion der Arylesterasen des Pankreas durch transkutane Messung der Zunahme an Fluoreszenz im Blut bestimmt. Die Fluoreszenz stammt in diesem Fall beispielsweise von enzymatisch freigesetztem Fluorescein, welches aus Fluorescein-Dilaureat stammt, das als Substart der Arylesterasen an den zu untersuchenden Probanden verabreicht werden kann. Ähnliche Substrate, ie zur Pankreas Funktionsbestimmung eingesetzt werden können, sind Fluoresz-markierte Triglyceridanaloga oder ein Nitrophenylester eines Fluoreszenz-markierten Alkylphosphonats. Eine nähere Beschreibung solcher Substrate findet sich in Scholze 1999, Analytical Biochemistry 276:72-80 oder Negre-Salvayre 1990, Lipids 25 (8): 428-434. Auf die darin offenbarten Substrate wird hiermit ausdrücklich Bezug genommen.

Die Offenbarung betrifft mithin auch ein Verfahren zur transkutanen Messung der Pankreasfunktion, insbesondere unter Verwendung eines Sensorpflasters (116) nach einem der vorhergehenden, ein Sensorpflaster (116) betreffenden Ausführungsformen und/oder eines Sensorsystems (114) nach einer der vorhergehenden, ein Sensorsystem (114) betreffenden Offenbarungen und/oder eines Kits (110) nach einem der Ansprüche 4 bis 13, wobei das Verfahren folgende Schritte umfasst:
- ein Sensorpflaster (116) wird auf eine Körperoberfläche aufgebracht, insbesondere aufgeklebt, wobei das Sensorpflaster (116) mindestens eine Strahlenquelle umfasst, wobei die Strahlenquelle eingerichtet ist, um die Körperoberfläche mit mindestens einem Abfragelichts (162) zu bestrahlen, wobei das Sensorpflaster (116) weiterhin einen Detektor (146) umfasst, wobei der Detektor (146) eingerichtet ist, um mindestens ein aus Richtung der Körperoberfläche eingestrahltes Antwortlicht (176) zu erfassen;
- mindestens zwei zeitlich begrenzte Messungen zu verschiedenen Zeitpunkten und/oder mindestens eine kontinuierliche Messung über einen Zeitraum hinweg werden durchgeführt, wobei das Antwortlicht (176) zu den verschiedenen Zeitpunkten und/oder über den Zeitraum hinweg erfasst wird; und
- aus einem zeitlichen Verlauf des Antwortlichts (176) wird auf einen zeitlichen Verlauf einer Konzentration einer Indikatorsubstanz (112) geschlossen, wobei die Indikatorsubstanz Fluorescein-Dilaurat, ein Fluoresz-markiertes Triglyceridanalogon oder ein Nitrophenylester eines Fluoreszenz-markierten Alkylphosphonats ist.

### Beispiele

Weitere Einzelheiten und Merkmale der Offenbarung ergeben sich aus der nachfolgenden Beschreibung bevorzugter Beispiele. Die Beispiele sind in den Figuren schematisch dargestellt. Gleiche Bezugszeichen bezeichnen dabei gleiche oder funktionsgleiche bzw. in ihren Funktionen einander entsprechende Elemente. Die Erfindung ist nicht auf die Beispiele beschränkt.

Im Einzelnen zeigt:
- Figur 1: ein Beispiel eines offenbarten Sensorsystems und Kits zur transkutanen Messung einer Organfunktion;
- Figuren 2A und 2B: ein Beispiel eines offenbarten Sensorpflasters in verschiedenen Darstellungen;
- Figur 3: ein Beispiel einer in dem Sensorpflaster einsetzbaren organischen Leuchtdiode;
- Figur 4: ein Beispiel einer in dem Sensorpflaster einsetzbaren organischen Solarzelle;
- Figur 5: einen Ablaufplan eines möglichen Beispiels eines offenbarten Verfahrens zur transkutanen Messung einer Organfunktion;
- Figuren 6A bis 6D: einen Nachweis von Fluoreszenz-markierten Inulin-Fraktionen im interstitiellen Gewebe;
- Figuren 7A bis 7D: Clearance Experimente mit F5 und F10 Inulin Fraktionen und Sinistrin.

### Beispiel 1: Messaufbauten

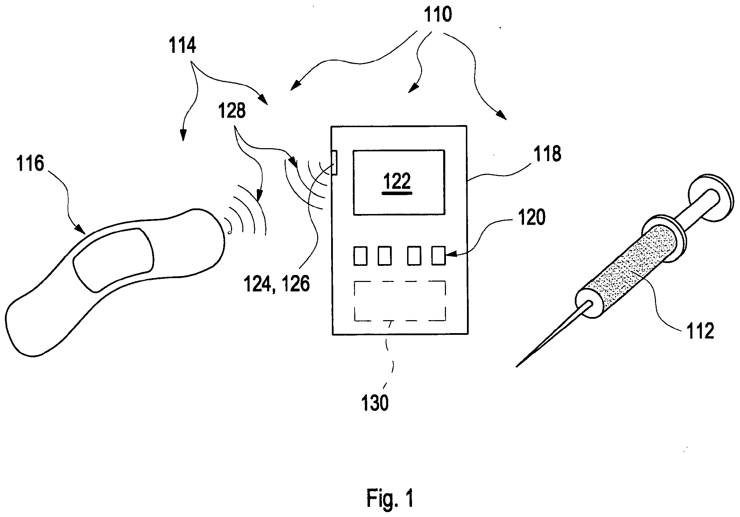

In Figur 1 ist ein Beispiel eines offenbarten Kits 110 zur transkutanen Messung einer Organfunktion stark schematisiert dargestellt. Das Kit 110 umfasst in diesem Beispiel eine Indikatorsubstanz 112, welche hier symbolisch als Inhalt einer Spritze dargestellt ist. Alternativ oder zusätzlich zu einer Injektion dieser Indikatorsubstanz 112 kommen jedoch auch andere Arten der Verabreichung in Betracht, beispielsweise orale, transdermale oder rektale Verabreichungen. Weiterhin kann auch auf körpereigene Indikatorsubstanzen zurückgegriffen werden. Entsprechend kann das Kit 110 geeignete Darreichungsformen für diese Indikatorsubstanz 112 umfassen, beispielsweise Spritzen, Ampullen, Tabletten, Beutel, Röhrchen oder Ähnliches.

Neben der Indikatorsubstanz 112 umfasst das Kit 110 in dem dargestellten Beispiel ein Sensorsystem 114 zur transkutanen Messung einer Organfunktion. Das Sensorsystem 114 umfasst ein Sensorpflaster 116 zur transkutanen Messung einer Organfunktion, welches in Figur 1 lediglich symbolisch angedeutet ist. Weiterhin umfasst das Sensorsystem 114 ein Lesegerät 118, welches ebenfalls stark schematisiert gezeigt ist. Das Lesegerät 118 kann beispielsweise ein oder mehrere Ein- und Ausgabemittel umfassen, welche in Figur 1 symbolisch in Form von Bedienelementen 120 dargestellt sind. Weiterhin kann das Lesegerät 118 ein oder mehrere Anzeigeelemente 122 umfassen, beispielsweise ein oder mehrere Displays, akustische Anzeigeelemente oder Ähnliches, beispielsweise um einem Benutzer Messergebnisse oder andere Informationen mitzuteilen.

Weiterhin kann das Lesegerät 118 eine oder mehrere Schnittstellen 124 umfassen, beispielsweise eine Hochfrequenz-Schnittstelle 126, zur Kommunikation mit dem Sensorpflaster 116. Alternativ oder zusätzlich können weitere Schnittstellen 124 vorgesehen sein, beispielsweise drahtgebundene Schnittstellen, beispielsweise ebenfalls zur Kommunikation mit dem Sensorpflaster 116 und/oder mit weiteren elektronischen Geräten, beispielsweise einem externen Computersystem. Eine drahtlose Kommunikation über elektromagnetische Hochfrequenzstrahlung ist in Figur 1 symbolisch mit der Bezugsziffer 128 bezeichnet. Diese Kommunikation 128 kann, wie in Figur 1 angedeutet, bidirektional stattfinden oder kann auch lediglich unidirektional erfolgen.

Weiterhin kann das Lesegerät 118, wie in Figur 1 angedeutet, eine Ansteuer- und Auswerteelektronik 130 umfassen. Diese Ansteuer- und Auswerteelektronik 130 kann beispielsweise ein oder mehrere elektronische Komponenten umfassen, beispielsweise ein Datenverarbeitungsgerät, einen oder mehrere flüchtige und/oder nicht-flüchtige Speicher und andere Komponenten.

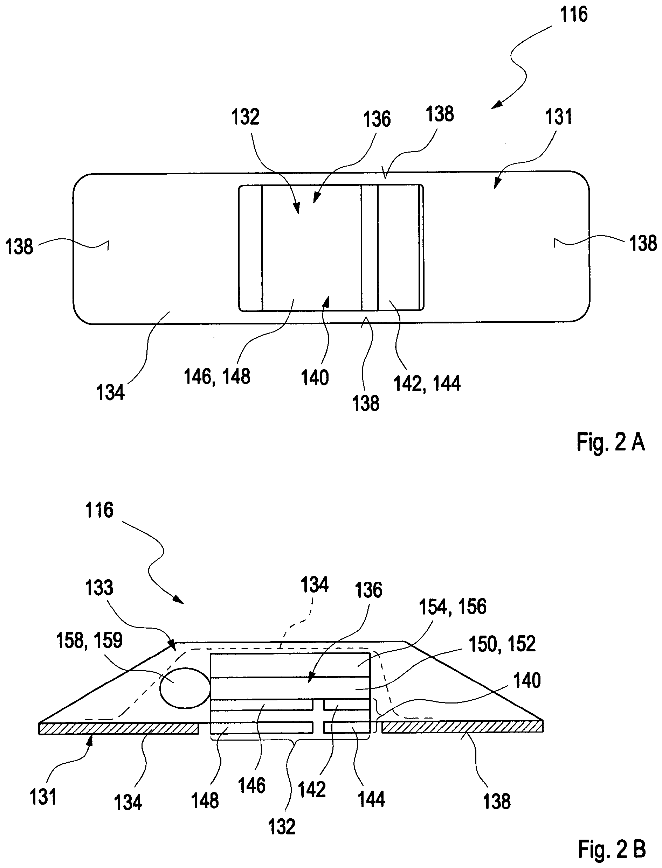

In den Figuren 2A und 2B ist eine schematische Darstellung möglicher Beispiele eines offenbarten Sensorpflasters 116 in verschiedenen Blickrichtungen dargestellt. Das Sensorpflaster 116 weist eine Vorderseite 131 auf, welche in einem Zustand, in dem das Sensorpflaster 116 auf eine Körperoberfläche (in den Figuren nicht dargestellt) aufgebracht ist, der Körperoberfläche zuweist, und eine von der Körperoberfläche weg weisende Rückseite 133. Dabei zeigt Figur 2A eine Draufsicht auf die Vorderseite 131 des Sensorpflasters 116, wohingegen Figur 2B stark schematisiert eine perspektivische Ansicht des Sensorpflasters 116 zeigt. In dieser perspektivischen Ansicht ist jedoch symbolisch, abweichend von der perspektivischen Darstellung, ein Schichtaufbau angedeutet. Die Vorderseite 131 ist in der Darstellung gemäß Figur 2B unten.

Wie aus der Draufsicht gemäß Figur 2A auf die Vorderseite 131 des Sensorpflasters 116 hervorgeht, weist das Sensorpflaster 116 ein flexibles Trägerelement 134 auf. Dieses flexible Trägerelement 134 kann beispielsweise lichtdicht ausgestaltet sein und kann als Träger für das eigentliche Sensormodul 136 dienen. Beispielsweise kann das flexible Trägerelement 134 in Form eines Rechteckigen, länglichen Streifens ausgestaltet sein, und kann beispielsweise ein Trägermaterial mit mindestens einem flexiblen Material und/oder einem Schichtaufbau derartiger flexibler Materialien umfassen. Beispielsweise können hier Kunststoffmaterialien, keramische Materialien, Papiermaterialien, Glasmaterialien oder Kombinationen der genannten und/oder anderer Materialien zum Einsatz kommen.

Das Trägerelement 134 soll derart flexibel ausgestaltet sein, dass sich dieses derart verformen lässt, dass eine Anpassung an die jeweilige Körperoberfläche, auf welcher die Messung stattfinden soll, möglich ist. Insofern ist der Begriff "flexibel" im Rahmen der vorliegenden Erfindung als "verformbar" auszulegen.

Das Trägerelement 134 kann, wie in Figur 2B durch die gestrichelte Linie angedeutet, das Sensormodul 136 auf der Rückseite 133 vollständig überdecken. Auch eine lediglich partielle Überdeckung ist jedoch grundsätzlich möglich, beispielsweise wenn das Sensormodul 136 zusätzlich (siehe unten) eine Solarzelle umfasst, mit einer Solarzellenfläche, die zur Rückseite 133 hin in weist.

Das Sensormodul 136 weist mindestens eine aktive Fläche 132 auf, welche der Vorderseite 131 und somit im applizierten Zustand des Sensorpflasters 116 der Körperoberfläche zuweist. Diese aktive Fläche 132 kann auch in Form von mehreren Einzelflächen ausgestaltet sein. Die aktive Fläche 132 kann beispielsweise eine oder mehrere lichtemittierende Flächen mindestens einer Lichtquelle 142, eine oder mehrere Detektorflächen mindestens eines Detektors 146, einen oder mehrere Filter 144, 148, optische Elemente, Schutzelemente oder andere Komponenten des Sensormoduls 136 und/oder Kombinationen der genannten Elemente und/oder anderer Elemente des Sensormoduls 136 umfassen.

Das Trägerelement 134 weist eine Haftoberfläche 138 auf, welche in dem Beispiel gemäß Figur 2A die aktive Fläche 132 vollständig umschließt. Die Haftoberfläche 138 kann beispielsweise mittels eines Klebstoffs als selbstklebende Haftoberfläche 138 ausgestaltet sein. Insbesondere kann diese Haftoberfläche 138 wiederum derart ausgestaltet sein, dass bei aufgeklebtem Sensorpflaster 116 kein Umgebungslicht zu dem Sensormodul 136 gelangen kann.

Das Sensormodul 136 weist in dem dargestellten Beispiel als, von der Vorderseite 131 her betrachtet, unterstes Element eines Schichtaufbaus eine optische Einheit 140 auf. Diese optische Einheit 140, deren Schichtaufbau beispielsweise in Figur 2B zu erkennen ist, umfasst in dem dargestellten Beispiel eine Lichtquelle 142, welche beispielsweise als organische lichtemittierende Diode (OLED) ausgestaltet ist. Auf dieser Lichtquelle 142 kann ein Anregungsfilter 144, beispielsweise eine Filterfolie, aufgebracht sein, so dass dieser Anregungsfilter 144 zur Körperoberfläche hin weist.

Weiterhin umfasst die optische Einheit 140 in dem dargestellten Beispiel eine Detektor 146, beispielsweise eine organische Solarzelle. Dieser Detektor 146 ist auf seiner der aktiven Fläche 132 zuweisenden Seite beispielsweise mit einem Antwortfilter 148 versehen, beispielsweise wiederum in Form einer auf den Detektor 146 aufgeklebten Filterfolie.

Wie aus Figur 2A und Figur 2B zu erkennen ist, sind sowohl die Lichtquelle 142 als auch der Detektor 146 als großflächige Bauelemente ausgestaltet, so dass der aktiven Fläche 132, welche unmittelbar auf der Körperoberfläche des Patienten aufliegt, jeweils eine große Fläche dieser Bauelemente zuweist. Beispielsweise können sowohl die Lichtquelle 142 als auch der Detektor 146 aktive Flächen aufweisen, welche der Körperoberfläche zuweisen, welche beispielsweise einige 10 mm² umfassen. Auch kleinere oder größere Flächen sind jedoch grundsätzlich möglich. Auf diese Weise ist sichergestellt, dass sowohl großflächig Abfragelicht auf die Körperoberfläche eingestrahlt wird, als auch großflächig Antwortlicht von der Körperoberfläche empfangen werden kann. Für derartig großflächige Bauelemente eignen sich organische Bauelemente besonders gut, da diese, beispielsweise im Gegensatz zu herkömmlichen anorganischen Halbleiterbauelementen, von Natur aus großflächig ausgestaltet sind.

In der nächsten Schichtebene, auf der der aktiven Fläche 132 abgewandten Seite der optischen Einheit 140, umfasst das Sensorpflaster 116 in dem dargestellten Beispiel eine elektronische Einheit 150. Alternativ oder zusätzlich zu dem in Figur 2B dargestellten Beispiel kann diese elektronische Einheit 150 jedoch auch auf andere Weise angeordnet sein, beispielsweise ganz oder teilweise neben der optischen Einheit 140. Der dargestellte Schichtaufbau lässt sich jedoch beispielsweise drucktechnisch besonders einfach realisieren und bewirkt kurze elektronische Übertragungswege sowie eine flache und kompakte Bauweise. Die elektronische Einheit 150 kann beispielsweise eine Ansteuerelektronik 152 zum Ansteuern und/oder Auswerten der optischen Einheit 140 umfassen. Beispielsweise können mittels dieser Ansteuerelektronik 152 die Lichtquelle 142 zur Emission von Abfragelicht und/oder der Detektor 146 zur Erfassung von Antwortlicht angeregt werden. Weiterhin kann die Ansteuerelektronik 152 auch ein oder mehrere Datenspeicher umfassen, um zumindest eine Zwischenspeicherung der Messergebnisse, die mittels des Detektors 146 gewonnen wurden, vorzunehmen. Verschiedene andere Ausgestaltungen sind möglich.

Weiterhin umfasst das Sensorpflaster 116 gemäß dem in den Figuren 2A und 2B dargestellten Beispiel eine Kommunikationseinheit 154, welche beispielsweise ganz oder teilweise als Schnittstelle 156 für eine Kommunikation mit dem Lesegerät 118 ausgestaltet sein kann. Diese Kommunikationseinheit 154 kann beispielsweise in RFID-Technologie ausgestaltet sein und/oder kann eine Hochfrequenzspule umfassen, um die in Figur 1 symbolisch mit Bezugsziffer 128 bezeichnete drahtlose Kommunikation mit dem Lesegerät 118 zu bewerkstelligen. Auch die Kommunikationseinheit 154 kann ganz oder teilweise von der Ansteuerelektronik 152 angesteuert werden und/oder kann über eine separate Ansteuerelektronik 152 verfügen.

Weiterhin umfasst das Sensorpflaster 116 in dem in Figur 2B dargestellten Beispiel eine elektrische Energiequelle 158 Während die Kommunikationseinheit 154, die elektronische Einheit 150 und die optische Einheit 140 in dem in den Figuren 2A und 2B dargestellten Beispiel in Schichtbauweise übereinander angeordnet sind, was jedoch ebenfalls nicht notwendigerweise der Fall sein muss, ist die elektrische Energiequelle 158 in Figur 2B neben diesem Schichtaufbau angeordnet. Alternativ oder zusätzlich kann die mindestens eine elektrische Energiequelle 158 jedoch auch vollständig oder teilweise in den Schichtaufbau der Einheiten 140, 150 und 154 integriert sein.

Die elektrische Energiequelle 158 kann beispielsweise eine gedruckte Batterie, beispielsweise eine gedruckte Polymerbatterie, umfassen. Die elektrische Energiequelle 158 kann eine oder mehrere der Einheiten 140, 150 und 154 mit elektrischer Energie versorgen. Wie oben dargestellt, kann das Sensorpflaster 116 jedoch, alternativ oder zusätzlich zu der mindestens einen elektrischen Energiequelle 158, auch eine oder mehrere Energieerzeugungsvorrichtungen umfassen, welche in Figur 2B symbolisch mit der Bezugsziffer 159 bezeichnet sind. Diese Energieerzeugungsvorrichtungen 159 können, wie in Figur 2B symbolisch angedeutet, gemeinsam mit der elektrischen Energiequelle 158 ausgestaltet sein, können jedoch auch ganz oder teilweise räumlich getrennt von dieser elektrischen Energiequelle 158 ausgebildet werden.

Beispielsweise kann die erforderliche elektrische Energie von außen eingestrahlt werden, wie dies bei üblicher Transpondertechnologie verwendet wird. Hierfür kann beispielsweise die Kommunikationseinheit 154 ihre für eine Kommunikation mit dem Lesegerät 118 erforderliche Energie aus den eingestrahlten elektromagnetischen Wellen empfangen. Alternativ oder zusätzlich kann die Energieerzeugungsvorrichtung 159 auch beispielsweise eine oder mehrere Solarzellen umfassen, beispielsweise wiederum eine oder mehrere organische Solarzellen. Diese mindestens eine Solarzelle kann dann beispielsweise mindestens eine Solarzellenfläche umfassen, welche der Rückseite 133 des Sensorpflasters 116 zuweist und welche vorzugsweise zumindest nicht vollständig von den Trägerelement 134 überdeckt ist, so dass eine Einstrahlung von Umgebungslicht, insbesondere Sonnenlicht, auf diese Solarzellenfläche möglich ist. Wiederum alternativ oder zusätzlich kann die Energieerzeugungsvorrichtung 159 ein oder mehrere thermoelektrische Wandler umfassen, beispielsweise ein oder mehrere Peltier- oder Seebeck-Elemente. Auch andere Ausgestaltungen sind möglich oder auch Kombinationen der genannten und/oder anderer Möglichkeiten der Ausgestaltung der Energieerzeugungsvorrichtung 159.

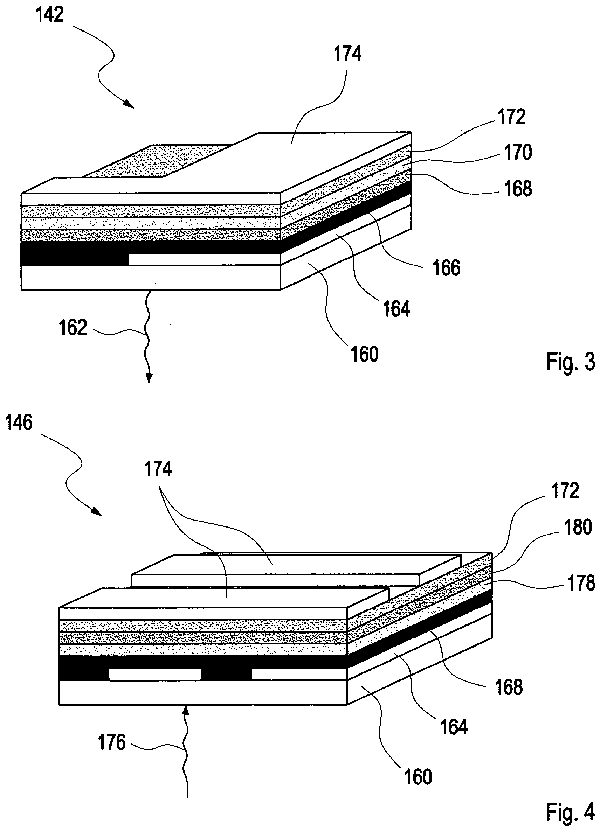

In den Figuren 3 und 4 sind mögliche Beispiele der Lichtquelle 142 (in Figur 3) bzw. des Detektors 146 (in Figur 4) in schematischer perspektivischer Darstellung gezeigt. Es sei darauf hingewiesen, dass es sich bei diesen Schichtaufbauten lediglich um Beispiele einer Vielzahl möglicher Schichtaufbauten handelt und dass auch andere als die dargestellten Materialien, andere Schichtabfolgen, andere Schichtdicken, andere Geometrien oder andere Arten der Erzeugung der Schichten verwendet werden können.

Die Lichtquelle 142 weist zunächst ein Substratmaterial 160 auf. Bei dem in Figur 3 dargestellten Beispiel ist dieses Substratmaterial 160 als transparentes Substratmaterial ausgestaltet, durch welches das von der Lichtquelle 142 erzeugte Abfragelicht 162 die Lichtquelle 142 verlassen kann. Insofern muss bei dem Schichtaufbau gemäß Figur 2B dieses Substratmaterial 160 der aktiven Fläche 132 zuweisen. Es sei darauf hingewiesen, dass, um beispielsweise die Schichtfolge der Lichtquelle 142 unmittelbar auf die übrigen Schichten des in Figur 2B dargestellten Schichtaufbaus aufdrucken zu können und/oder unmittelbar auf das Trägerelement 134 aufdrucken zu können, auf das Substratmaterial 160 auch verzichtet werden kann oder dass dieses Substratmaterial 160 durch eine andere Art von transparentem Material ersetzt werden kann. Derartige Aufbauten werden häufig auch als inverse Aufbauten bezeichnet, da bei derartigen Aufbauten die Schichtfolge der Lichtquelle 142 nicht auch in der dargestellten Reihenfolge auf das Substratmaterial 160 aufgebracht wird, sondern in umgekehrter Reihenfolge. Auch die Bezeichnung "upside down"-Schichtaufbau findet sich diesbezüglich. Auf dem transparenten Substratmaterial 160, welches beispielsweise ein Glas, beispielsweise ein dünnes, flexibles Glas, oder wahlweise ein transparentes Kunststoffmaterial oder eine Kombination dieser und/oder anderer Materialien umfassen kann, wird eine transparente Anode 164 aufgebracht. Wahlweise kann auch eine andere Elektrode als die Anode als transparente Elektrode ausgestaltet sein. Beispielsweise kann als transparentes Anodenmaterial Indium-Zinn-Oxid (ITO) verwendet werden, beispielsweise mit einer Schichtdicke von 30 bis 80 nm, beispielsweise 50 nm.

Auf diese transparente Anode 164 kann eine Barriereschicht 166 aufgebracht werden, welche auch als Lochinjektionsschicht ausgestaltet sein kann. Beispielsweise kann es sich hierbei um eine Oxidschicht handeln, mit einer Dicke im Bereich von wenigen Nanometern, beispielsweise 10 nm. Für einen möglichen Aufbau einer derartigen Lochinjektionsschicht kann auf die oben beschriebene Veröffentlichung von A. Pais et al. verwiesen werden.

Auf die Barriereschicht 166 ist eine dünne Schicht eines Lochtransportmaterials 168 aufgebracht. Dieses Lochtransportmaterial 168, welches besonders hohe Beweglichkeiten für positive Ladungsträger, beispielsweise Radikal-Kationen, aufweist, kann beispielsweise eine Schicht von wenigen Nanometern, beispielsweise 10 bis 50 nm, eines N,N'-diphenyl-N,N'-bis(1-naphthyl)(1,1'-biphenyl)-4,4'diamin (NPB) sein. Auch andere Lochtransportmaterialien oder Kombinationen mehrerer Schichten verschiedener Lochtransportmaterialien sind einsetzbar.

Auf dem Lochtransportmaterial 168 ist in dem in Figur 3 dargestellten Beispiel eine Schicht eines Emittermaterials 170 aufgebracht. In diesem Emittermaterial 170 erfolgt die Erzeugung der Photonen des Abfragelichts 162, indem dort positive und negative Ladungsträger rekombinieren und/oder Exziton-Paare abreagieren und dabei Photonen emittieren. Beispielsweise kann dieses Emittermaterial 170 eine Schicht von wenigen Nanometern, beispielsweise 10 bis 50 nm, eines tris(8-hydroxyquinolin)aluminium (Alq) umfassen. Auch andere Arten von Emittermaterialien oder Kombinationen verschiedener Emittermaterialien sind einsetzbar.

Auf das Emittermaterial 170 ist in dem dargestellten Beispiel eine Schicht eines Elektroninjektionsmaterials aufgebracht, welches eine Elektroneninjektion in das Emittermaterial 170 oder eines auf das Emittermaterial 170 aufgebrachten Elektrontransportmaterials (in Figur 3 nicht dargestellt) begünstigt. Beispielsweise kann dieses Elektroninjektionsmaterial 172 eine dünne Schicht eines Fluorids umfassen, beispielsweise Lithiumfluorid, beispielsweise in einer Schichtdicke von 0,5 bis 2 nm, insbesondere 1 nm. Auf dieses Elektroninjektionsmaterial 172 wird dann eine Kathode 174 aufgebracht, aus welcher Elektronen in den organischen Schichtaufbau injiziert werden.

Beispielsweise kann eine Aluminium-Kathode 174 mit einer Schichtdicke von 50 bis 200 nm, beispielsweise 100 nm, verwendet werden. Auch andere Elektrodenmaterialien sind grundsätzlich einsetzbar. Wird ein inverser Schichtaufbau verwendet, bei welchem die Emission des Abfragelichts 162 durch die Kathode 174 erfolgen muss, beispielsweise aufgrund der oben dargestellten Druckproblematik, so kann auch die Kathode 174, alternativ oder zusätzlich zur Anode 164, transparent ausgestaltet sein. Dies kann beispielsweise dadurch erfolgen, dass dünne Metallschichten verwendet werden, gegebenenfalls in Kombination mit transparenten Elektrodenmaterialien wie beispielsweise wiederum ITO.

Weiterhin ist in Figur 3 angedeutet, dass die Elektroden 164, 174 gegebenenfalls geeignet strukturiert sein können, um eine Kontaktierung dieser Elektroden 164, 174 zu ermöglichen.

Bei dem in Figur 3 dargestellten Beispiel handelt es sich um ein Beispiel einer Lichtquelle 142, bei welchem die aktiven Schichten vollständig aus niedermolekularen organischen Materialien hergestellt sind. Derartige niedermolekulare organische Materialien werden üblicherweise aus der Gasphase abgeschieden. Auch eine Flüssigphasenabscheidung ist jedoch grundsätzlich möglich. Es sei darauf hingewiesen, dass auch andere Materialien eingesetzt werden können und/oder andere Abscheidetechniken, beispielsweise Polymermaterialien, welche beispielsweise durch einen nasschemischen Prozess aufgebracht werden können. In letzterem Fall ist insbesondere ein Druckprozess oder ein Verfahren, bei welchem mehrere Druckprozesse eingesetzt werden, von Vorteil.

In Figur 4 ist, ebenfalls lediglich beispielhaft, ein Beispiel eines Detektors 146 in einer zu Figur 3 analogen Darstellung gezeigt. Wiederum sei darauf hingewiesen, dass auch andere Materialien, andere Schichtkombinationen, insbesondere inverse Aufbauten, Aufbauten mit zusätzlichen Schichten oder andere Arten von Modifikationen des gezeigten Schichtaufbaus möglich sind.

Der Detektor 146 in Figur 4 ist als organische Photodiode aufgebaut. Ausgangspunkt in dem dargestellten Beispiel ist wiederum ein Substratmaterial 160, was beispielsweise wiederum transparent ausgestaltet sein kann, so dass Antwortlicht, welches in Figur 4 mit der Bezugsziffer 176 bezeichnet ist, durch dieses Substratmaterial 160 in den Detektor 146 gelangen kann. Es sei wiederum darauf hingewiesen, dass im Rahmen der vorliegenden Erfindung auch inverse Aufbauten verwendet werden können, also Aufbauten, bei welchen das Antwortlicht 176 durch eine transparente Deck-elektrode (also in Figur 4 von oben her) in den Detektor 146 gelangen kann, ohne das Substratmaterial 160 zu durchdringen. Ein derartiger Aufbau wäre beispielsweise im Rahmen eines Druckverfahrens für den Einsatz in einem Sensorpflaster gemäß Figur 2B bevorzugt, bei welchem beispielsweise die in Figur 4 gezeigte Schichtfolge in inverser Reihenfolge auf das in Figur 2B dargestellte lichtundurchlässige Trägerelement 134 aufgedruckt würde. Der Lichteintritt des Antwortlichts 176 könnte dann entweder über eine transparente Kathode oder über eine transparente Anode erfolgen, welche auf der dem Trägerelement 134 abgewandten, der aktiven Fläche 132 zuweisenden Seite des Schichtaufbaus angeordnet wäre. Insofern gilt das für die organische Lichtquelle 142 gemäß Figur 3 Gesagte für den Detektor 146 analog.

Bei dem beispielhaften Schichtaufbau gemäß Figur 4 ist auf das transparente Substratmaterial 160 eine transparente Anode 164 aufgebracht, welche beispielsweise wiederum strukturiertes ITO umfassen kann, welches beispielsweise auf einem dünnen Glassubstrat 160 oder einem dünnen Kunststoffsubstrat 160 aufgebracht sein kann.

Auf das ITO der Anode 164 wird eine Lochtransportschicht aufgebracht, welche beispielsweise eine wenige 10 nm dicke Schicht, beispielsweise eine 50 nm dicke Schicht, Poly(3,4-ethylenedioxythiophen):Polysytrensulphonat (PEDOT:PSS) umfasst. Diese Schicht erfüllt beispielsweise ähnliche Funktionen wie das Lochtransportmaterial 168 gemäß Figur 3, so dass in Figur 4 ebenfalls die Bezugsziffer 168 für diese Lochtransportschicht verwendet wurde.

Auf diese Lochtransportschicht 168 ist in dem dargestellten Beispiel gemäß Figur 4 ein Doppelschichtsystem eines Akzeptor-Donator-Systems aufgebracht, welches Kupferphthalocyanin 178 und das Buckminsterfulleren C₆₀ 180 umfasst. Auch ein gemischtes System, in welchem diese Schichten 178, 180 beispielsweise durchmischt sind, ist denkbar. Während das Funktionsprinzip der organischen Leuchtdiode gemäß Figur 3 auf einer Erzeugung von Photonen bei einer Rekombination von Elektron-Loch-Paaren (bzw. deren organischen Äquivalenten) basiert, basiert das Funktionsprinzip der organischen Photodiode gemäß Figur 4 auf dem umgekehrten Effekt, bei welchem in das Bauelement eintretende Photonen Elektron-Loch-Paare (bzw. deren organische Äquivalente) erzeugen. Auf die C₆₀-Schicht 180 ist schließlich eine optionale LiF-Schicht 172 und eine strukturierte Kathode 174, beispielsweise eine Aluminium-Kathode, aufgebracht, ähnlich zum Aufbau gemäß Figur 3.

Für weitere Einzelheiten der möglichen, im Rahmen der vorliegenden Erfindung einsetzbaren Ausführungsbeispiele kann auf die oben beschriebene Veröffentlichung von A. Pais et al. verwiesen werden.

Es sei weiterhin darauf hingewiesen, dass die spektralen Eigenschaften der Bauelemente gemäß den Figuren 3 und 4 sich einfach an die jeweiligen Erfordernisse des Sensorpflasters 116 anpassen lassen. So kann beispielsweise das Abfragelicht 162 der Lichtquelle 142 an die jeweiligen Erfordernisse der Indikatorsubstanz 112 bzw. eines in dieser Indikatorsubstanz 112 enthaltenen Markers angepasst werden. Das dargestellte Bauelement mit Alq als Emittermaterial emittiert beispielsweise im grünen Spektralbereich. Es lassen sich jedoch Bauelemente herstellen, beispielsweise durch Dotierung des Emittermaterials mit geeigneten Farbstoffen und/oder durch Verwendung anderer Emittermaterialien, welche in anderen Spektralbereichen emittieren. Beispielsweise existieren zahlreiche organische Leuchtdioden, welche im kurzwelligen sichtbaren Spektralbereich emittieren, also beispielsweise im blauen Spektralbereich bis hin in den nahen und ultravioletten Spektralbereich. Auf diese Weise kann das Abfragelicht 162 beispielsweise an die jeweiligen Absorptionscharakteristika der Indikatorsubstanz 112 bzw. eines Markers dieser Indikatorsubstanz 112 angepasst werden. Beispielsweise existieren Emittermaterialien, welche im blauen Spektralbereich emittieren. Beispielsweise emittieren verschiedene Fluorenverbindungen als Polymermaterialien im blauen Spektralbereich. Bei den niedermolekularen Emittermaterialien sind beispielsweise Spiroverbindungen als mögliche Emitter im blauen Spektralbereich zu nennen. Verschiedene andere Ausgestaltungen sowie Kombinationen verschiedener Emittermaterialien sind möglich.

Analog lassen sich auch die spektralen Eigenschaften des Detektors 146 an das nachzuweisende Antwortlicht 176 anpassen, so dass eine optimale Signalerzeugung erfolgen kann. Dies kann beispielsweise dadurch erfolgen, dass ein von dem in Figur 4 dargestellten Donator-Akzeptor-System verschiedenes Donator-Akzeptor-System verwendet wird. Verschiedene Ausgestaltungen sind möglich. Auch ist es beispielsweise möglich, mehrere Lichtquellen 142 mit unterschiedlichen spektralen Eigenschaften und/oder mehrere Detektoren 146 mit unterschiedlichen Absorptionscharakteristika zu verwenden, so dass auch eine gleichzeitige Messung in mehreren Spektralbereichen erfolgen kann.

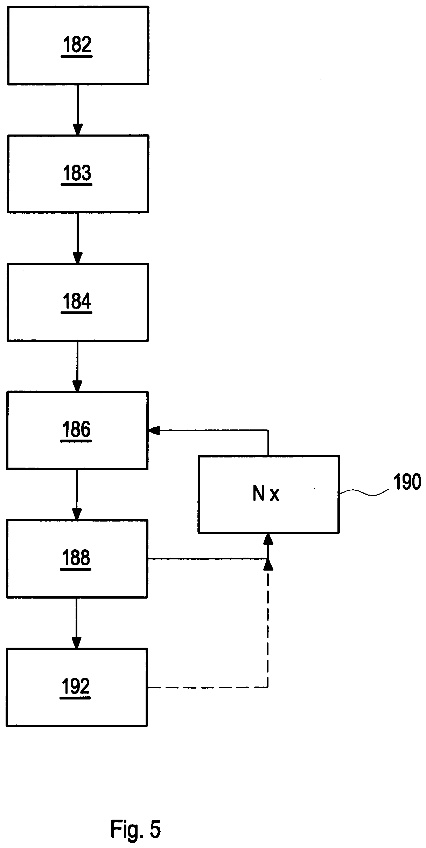

In Figur 5 ist schließlich ein Beispiel eines möglichen offenbarten Verfahrens zur transkutanen Messung einer Organfunktion als stark schematisierter Ablaufplan gezeigt.

Das Verfahren beginnt in Schritt 182 mit dem Aufbringen eines Sensorpflasters 116 auf eine Körperoberfläche eines menschlichen oder tierischen Patienten. Dies kann beispielsweise dadurch erfolgen, dass die Haftoberfläche 138, welche als selbstklebende Haftoberfläche ausgestaltet sein kann, auf die Körperoberfläche aufgeklebt wird.

Auf den Verfahrensschritt 182 folgt optional ein Schritt einer Nullwertmessung, der in Figur 5 mit der Bezugsziffer 183 bezeichnet ist. Dieser Verfahrensschritt 183 dient dem Zweck, Signale des Sensorpflasters 116 vor Einbringen der Indikatorsubstanz 112 zu bestimmen. Dies kann beispielsweise dem Zweck dienen, elektronische Offsets, Hintergrundsignale oder ähnliches zu beseitigen, und/oder um eine Lage der Koordinatenachsen festzulegen. Auch für andere Zwecke lassen sich die Ergebnisse der Nullwertmessung 183 einsetzen. Die Nullwertmessung 183 kann beispielsweise dadurch erfolgen, dass, ohne dass die Indikatorsubstanz 112 in den Körper eingebracht wurde, der unten beschriebene Schritt 186 einer Detektion durchgeführt wird. Auch eine mehrfache Durchführung dieses Verfahrensschritts 186 ist möglich. Weiterhin ist auch eine Durchführung zusätzlicher Verfahrensschritte möglich, beispielsweise der unten ebenfalls beschriebene Schritt 188 des Abspeicherns von Informationen, beispielsweise ein Abspeichern der Ergebnisse der Nullwertmessung 183.

Anschließend folgt in dem in Figur 5 dargestellten Beispiel des offenbarten Verfahrens ein Verfahrensschritt 184, in welchem die Indikatorsubstanz 112 in den Körper des Patienten eingebracht wird. Dieses Einbringen kann, wie oben dargestellt, beispielsweise durch orale Einnahme, durch Injektion oder Ähnliches erfolgen. Es sei darauf hingewiesen, dass dieser Verfahrensschritt 182 nicht notwendigerweise Bestandteil des Verfahrens sein muss, da beispielsweise auch endogene Indikatorsubstanzen 112 verwendet werden können, welche ohnehin im Körper vorhanden sind und deren Zufuhr beispielsweise unterbrochen werden kann oder deren Neuerzeugung blockiert werden kann. Verschiedene Ausgestaltungen sind denkbar.

In Verfahrensschritt 186 erfolgt eine Detektion einer Konzentration der Indikatorsubstanz 112 in einem Körpergewebe und/oder einer Körperflüssigkeit des Patienten durch eine transkutane Messung. Beispielsweise kann es sich um eine Messung in interstitieller Flüssigkeit handeln.

Zum Zweck dieser Detektion 186 wird mittels der Lichtquelle 142 Abfragelicht 162 durch die Körperoberfläche in das Körpergewebe bzw. die Körperflüssigkeit eingestrahlt, dort eine entsprechende Wechselwirkung mit der Indikatorsubstanz 112 bzw. einem Marker dieser Indikatorsubstanz 112 hervorgerufen, so dass das Antwortlicht 176 entsteht. Dieses Antwortlicht 176 wird mittels des Detektors 146 aufgenommen. Damit entsteht ein erstes Messsignal, beispielsweise in Form eines Messwertpaares, welches beispielsweise den Zeitpunkt der Messung bzw. Detektion 186, den gemessenen Wert des Antwortlichts 176 (beispielsweise eine Intensität und/oder eine zu dieser Intensität korrelierende Größe, beispielsweise eine Photospannung) beinhalten kann. Auch weitere Daten können in diesem Messwertpaar enthalten sein, beispielsweise eine Leuchtdichte der Lichtquelle 142 oder eine zu dieser Leuchtdichte korrelierende Größe, beispielsweise ein Strom durch die Lichtquelle 142.

Diese Messergebnisse werden in Schritt 188 abgespeichert. Dieses Abspeichern kann beispielsweise in einem internen Speicher des Sensorpflasters 116 erfolgen oder kann, alternativ oder zusätzlich, auch in einem Speicher des Lesegeräts 118 erfolgen. Beispielsweise kann das Sensorpflaster 116, insbesondere die elektronische Einheit 150 und/oder die Kommunikationseinheit 154, einen flüchtigen oder nicht-flüchtigen Speicher, beispielsweise einen Flash-Speicher, umfassen.

Anschließend können die Verfahrensschritte 186 und 188, wie durch die Bezugsziffer 190 in Figur 5 angedeutet, wiederholt werden. Auch die Auswertung 192, welche unten näher erläutert wird, kann ganz oder teilweise Bestandteil der Wiederholung 190 sein, was in Figur 5 durch die gestrichelte Linie angedeutet ist. Die Wiederholung 190 kann auch derart erfolgen, dass zwischen den einzelnen Wiederholungen eine vorgegebene Zeit abgewartet wird und/oder dass die Wiederholungen zu vorgegebenen Zeitpunkten stattfinden. Auf diese Weise kann durch eine N-fache Wiederholung eine Messserie aufgenommen werden, bei welcher die Detektion 186 über einen gewissen Zeitraum hinweg kontinuierlich oder diskontinuierlich erfolgt, beispielsweise in festen oder variablen Zeitabständen.

Anschließend erfolgt in Verfahrensschritt 192 optional eine Auswertung. Diese Auswertung 192 kann auf unterschiedliche Weisen und zu unterschiedlichen Graden erfolgen. Beispielsweise kann die Auswertung ganz oder teilweise bereits im Sensorpflaster 116, beispielsweise in der elektronischen Einheit 150, insbesondere der Ansteuerelektronik 152, vorgenommen werden. Alternativ oder zusätzlich kann jedoch auch eine Auswertung im Lesegerät 118, dort insbesondere in der Ansteuer- und Auswerteelektronik 130, und/oder in einem separaten Computersystem, welches beispielsweise mit dem Lesegerät 118 in Verbindung stehen kann, erfolgen. Auch eine Wiederholung ist möglich.

Die Auswertung kann beispielsweise in einer Glättung der Messergebnisse, einer Filterung der Messergebnisse, einer Anpassung von Messkurven (beispielsweise um eine Halbwertszeit zu bestimmen), in einer grafischen Darstellung oder Ähnlichem bestehen. Auch eine Kombination der genannten Schritte und/oder anderer Auswertungsschritte ist denkbar. Als Ergebnis der Auswertung 192 kann beispielsweise die Halbwertszeit und/oder eine renale Clearance der Indikatorsubstanz 112 ermittelt werden. Auch andere Parameter sind denkbar.

### Beispiel 2: Eigenschaften von definierten Inulingemischen

Definierte Inulingemische mit 3 bis 8 (F5) oder 11 bis 15 (F10) Zuckermonomeren wurden aus dem Rohstoff Inulin durch Verdau mit einer Inulinase und anschließender chromatographischen Trennung in einzelne Fraktionen gewonnen.

Die chromatographisch aufgetrennten Fraktionen F5 und F10 wurden mit Fluoreszeinisothiocyanat (FITC) zu FITC-F5 und FITC-F10 derivatisiert. FITC-F10 wurden an Ratten intravenös verabreicht. Die interstitielle Fluoreszenz des FITC bei einer Anregungswellenlänge von 485-520 nm gemessen wurde bestimmt. Als Kontrolle wurde die Fluoreszenz im Serum gemessen.

Es fiel auf, dass mit einer Reduktion der Zuckerreste die renale Exkretionsrate graduell abnahm, mit Halbwertszeiten von 25,98 +/- 2,66 Min. für FITC-F10 und 30,3 +/- 2,2 Min. für FITC-F5 verglichen mit einer Halbwertszeit von 25,02 +/- 1,67 Min. für Sinistrin bzw. 23,04 +/- 1,02 Min. für FITC-Sinistrin und 22,0 +/- 0,8 Min. für die unmarkierte Inulin-F5-Fraktion.

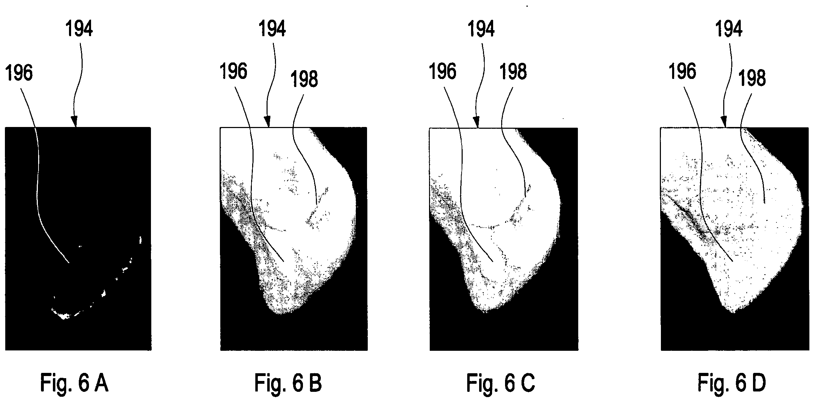

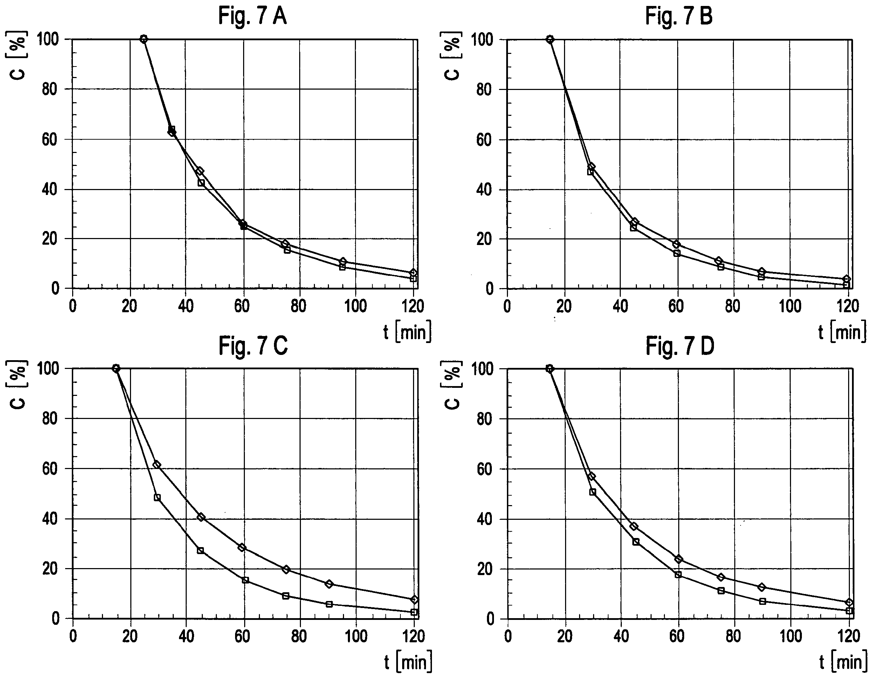

Die Erhöhung der Halbwertszeit kann zumindest teilweise durch eine Erhöhung der lipophilen Eigenschaften der Moleküle nach Fluoreszenzmarkierung erklärt werden. Die Markierungseffizienz für die F10- und F5-Fraktionen war zudem so, dass die Fluoreszenz auch noch nach drastischer Dosisreduktion um Faktor 10 oder mehr bestimmt werden konnte. Die Ergebnisse sind in den Figuren 6A bis 6D und 7A bis 7D graphisch dargestellt.

In den Figuren 6A bis 6D sind Aufnahmen eines Rattenohrs 194 dargestellt, welche mit einem Kleintier-Imager vom Typ CRI-Maestro gewonnen wurden. Die Aufnahmezeiten betragen 0 min (Fig. 6A), 1 min (Fig. 6B), 10 min (Fig. 6C), und 120 min (Fig. 6D). Die in den Figuren hell zu erkennenden, fluoreszierenden Areale entsprechen dem interstitiellen Raum 196 im Gewebe. Bereiche ohne Fluoreszenz markieren den Verlauf von Blutgefäßen 198. FITC-markierte Polyfructosane können also grundsätzlich transkutan im Interstitium gemessen werden.

Die Figuren 7A bis 7D zeigen Clearance-Experimente mit FITC-markierten Polyfructosanen, die enzymatisch bzw. fluorometrisch in Plasmaproben gemessen wurden. In allen Figuren ist die relative Konzentration c in Prozent gegen die Zeit t in Minuten aufgetragen. Fig. 7A zeigt die Abnahme der relativen Konzentrationen von markiertem (FITC-S, Messwerte als Rauten dargestellt) und nicht-markiertem Sinistrin (S, Messwerte als Quadrate dargestellt) über die Zeit. FITC-S wurde als Bolus von 250mg/kg, S als Bolus von 750mg/kg Körpergewicht an Ratten verabreicht. Die Halbwertszeiten für FITC-S und S betragen 23,9+/-1,4 min bzw. 22,8+/-1,4 min.

In den Figuren 7B und 7C wird die Abnahme der relativen Konzentration für FITC-F10 (Fig. 7B) und FITC-F5 (Fig. 7C) über die Zeit dargestellt und mit der von S verglichen (Messwerte für FITC-F10 bzw. FITC-F5 sind als Rauten, die für S als Quadrate dargestellt). S wurde als Bolus von 750mg/kg Körpergewicht, FITC-F10 als Bolus von 12mg/kg Körpergewicht, FITC-F5 als Bolus von 14mg/kg Körpergewicht an Ratten verabreicht. Die Halbwertszeiten in Fig. 7B betragen 24,5+/-1,4 min für FITC-F10 und 19,9+/-0,9 min für S, in Fig. 7C 30,0+/-0,6 min für FITC-F5 und 21,0+/-0,1 min für S.

Fig. 7D zeigt einen Vergleich der Abnahme der relativen Konzentrationen für markierte (FITC-F5, Messwerte als Rauten dargestellt) und nicht-markierte F5 Inuline (F5, Messwerte als Quadrate dargestellt) über die Zeit. FITC-F5 wurde als Bolus von 14mg/kg Körpergewicht, F5 als Bolus von 750mg/kg Körpergewicht verabreicht. Die Halbwertszeiten betragen 29,5+/-1,5 min für FITC-F5 und 21,9+/-0,6 min für F5. Aus den geringeren Bolusgaben für FITC-F10 und FITC-F5 im Vergleich zu FITC-S, ist auf eine deutlich bessere Markierungseffizienz zu schließen. Die erhöhten Halbwertszeiten für FITC-F10 und FITC-F5 lassen sich durch den stärkeren lipophilen Einfluss der FITC Gruppe auf die lipophilen Eigenschaften des Gesamtmoleküls erklären.

## Patentansprüche

1. Verwendung einer Fluoreszenz-markierten Indikatorsubstanz zur Herstellung eines Diagnostikums zur Bestimmung der glomerulären Filtrationsrate (GFR) am Körper eines menschlichen oder tierischen Patienten, wobei die Indikatorsubstanz ein Gemisch aus Inulinen ist, die von 11 bis 15 oder 3 bis 8 Fructoseeinheiten aufweisen, wobei die Inuline mit einem fluoreszierenden Marker gekoppelt sind.

2. Verwendung nach Anspruch 1, wobei die Indikatorsubstanz ein Gemisch aus Inulinen ist, die von 3 bis 8 Fructoseeinheiten aufweisen, wobei die Inuline mit einem fluoreszierenden Marker gekoppelt sind.

3. Verwendung nach Anspruch 1 oder 2, wobei die GFR bestimmt wird zur Diagnose einer bestehenden Nierenfunktionsstörung, zur Bestimmung des Risikos für zukünftige Nierenfunktionsstörungen, zum Überwachen ("Monitoring") bei Erkrankungen, therapeutischen Eingriffen oder Therapien, die Nierenfunktionsstörungen verursachen können, oder zur Bestimmung der individuellen Dosis für Arzneimittel, die über die Niere ausgeschieden werden.

4. Kit (110) zur transkutanen Messung einer Organfunktion, insbesondere einer Nierenfunktion, umfassend
(a) mindestens eine Indikatorsubstanz (112), wobei die Indikatorsubstanz (112) in einen Körper einbringbar ist, wobei ein zeitlicher Konzentrationsverlauf der Indikatorsubstanz (112) in dem Körper, insbesondere in einem Körpergewebe und/oder einer Körperflüssigkeit, als Indikator für die Organfunktion einsetzbar ist, wobei die Indikatorsubstanz (112) mindestens einen Marker umfasst, wobei der Marker eingerichtet ist, um bei Einstrahlung des mindestens einen Abfragelichts (162) der Strahlenquelle des Sensorpflasters (116) das mindestens eine Antwortlicht (176) auszusenden; und
(b) mindestens ein Sensorpflaster (116) umfassend mindestens ein flexibles Trägerelement (134) mit mindestens einer auf eine Körperoberfläche aufklebbaren Haftoberfläche (138), weiterhin umfassend mindestens eine Strahlenquelle, insbesondere eine Lichtquelle (142), wobei die Strahlenquelle eingerichtet ist, um die Körperoberfläche mit mindestens einem Abfragelicht (162) zu bestrahlen, wobei das Abfragelicht ein für die Detektion der Indikatorsubstanz verwendbares Licht ist, weiterhin umfassend mindestens einen Detektor (146), wobei der Detektor (146) eingerichtet ist, um mindestens ein aus Richtung der Körperoberfläche eingestrahltes Antwortlicht (176) zu erfassen,
wobei die Indikatorsubstanz ein Gemisch aus Inulinen ist, die von 11 bis 15 oder 3 bis 8 Fructoseeinheiten aufweisen, wobei die Inuline mit einem fluoreszierenden Marker gekoppelt sind.

5. Kit (110) nach Anspruch 4, wobei die Indikatorsubstanz ein Gemisch aus Inulinen ist, die von 3 bis 8 Fructoseeinheiten aufweisen, wobei die Inuline mit einem fluoreszierenden Marker gekoppelt sind.

6. Kit (110) nach Anspruch 4 oder 5, wobei die mindestens eine Strahlenquelle im Sensorpflaster (116) mindestens eine Lichtquelle (142) mit einem organischen lichtemittierenden Material (170) umfasst, insbesondere einen organische lichtemittierende Diode.

7. Kit (110) nach einem der Ansprüche 4 bis 6, wobei der mindestens eine Detektor (146) im Sensorpflaster (116) mindestens einen Detektor (146) mit mindestens einem organischen halbleitenden Material (168, 178, 180) umfasst, insbesondere einen organischen Photodetektor.

8. Kit (110) nach einem der Ansprüche 4 bis 7, wobei das Sensorpflaster (116) weiterhin mindestens eine Ansteuerelektronik (152) umfasst, wobei besagte Ansteuerelektronik (152) des Sensorpflasters (116) mindestens ein organisches Bauelement umfasst, insbesondere eine organische Leiterbahn und/oder einen organischen Feldeffekttransistor.

9. Kit (110) nach Anspruch 8, wobei die Ansteuerelektronik (152) des Sensorpflasters (116) eingerichtet ist, um eine zeitaufgelöste Messung des Sensorpflasters (116) zu steuern.

10. Kit (110) nach einem der Ansprüche 4 bis 9, wobei die Haftoberfläche (138) des Sensorpflasters (116) den Detektor (146) seitlich umschließt, wobei bei auf die Körperoberfläche aufgeklebtem Sensorpflaster (116) verhindert wird, dass Umgebungslicht zu dem Detektor (146) gelangen kann.

11. Kit (110) nach einem der Ansprüche 4 bis 10, wobei das Sensorpflaster (116) in einer Schichtbauweise hergestellt ist und mindestens zwei verschiedene Schichtebenen aufweist.

12. Kit (110) nach einem der Ansprüche 4 bis 11, weiterhin umfassend mindestens ein Lesegerät (118), wobei das Lesegerät (118) eingerichtet ist, um mit dem Sensorpflaster (116) zusammenzuwirken, insbesondere um eine Messung der Organfunktion mittels des Sensorpflasters (116) zu initiieren und/oder auszulesen.

13. Kit (110) nach Anspruch 12, wobei das Lesegerät (118) mindestens einen Hochfrequenz-Transmitter (126) umfasst, welcher eingerichtet ist, um einen Hochfrequenzpuls zu emittieren, wobei das Sensorpflaster (116) eingerichtet ist, um bei Empfang des Hochfrequenzpulses eine Messung mit Aussendung des Abfragelichts (162) und Empfang des Antwortlichts (176) zu starten.

## Claims

1. Use of a fluorescently labelled indicator substance for the production of a diagnostic aid for determining the glomerular filtration rate (GFR) on the body of a human or animal patient, wherein the indicator substance is a mixture of inulins comprising from 11 to 15 or from 3 to 8 fructose units, wherein the inulins are coupled to a fluorescent label.

2. Use according to Claim 1, wherein the indicator substance is a mixture of inulins comprising from 3 to 8 fructose units, wherein the inulins are coupled to a fluorescent label.

3. Use according to Claim 1 or 2, wherein the GFR is determined for the purpose of diagnosing an existing kidney dysfunction, for the purpose of determining the risk of future kidney dysfunctions, for the purpose of monitoring in the case of diseases, therapeutic interventions or therapies which can cause kidney dysfunctions, or for the purpose of determining the individual dose for medicaments that are excreted via the kidneys.

4. Kit (110) for the transcutaneous measurement of an organ function, more particularly of a kidney function, comprising
(a) at least one indicator substance (112), wherein the indicator substance (112) can be introduced into a body, wherein a temporal concentration profile of the indicator substance (112) in the body, more particularly in a body tissue and/or a body fluid, can be used as an indicator for the organ function, wherein the indicator substance (112) comprises at least one label, wherein the label is designed to emit the at least one response light (176) upon incidence of the at least one interrogation light (162) from the radiation source of the sensor plaster (116); and
(b) at least one sensor plaster (116) comprising at least one flexible carrier element (134) having at least one adhesive surface (138) which can be stuck onto a body surface, furthermore comprising at least one radiation source, more particularly a light source (142), wherein the radiation source is designed to irradiate the body surface with at least one interrogation light (162), wherein the interrogation light is a light which can be used for the detection of the indicator substance, furthermore comprising at least one detector (146), wherein the detector (146) is designed to detect at least one response light (176) incident from the direction of the body surface,
wherein the indicator substance is a mixture of inulins comprising from 11 to 15 or from 3 to 8 fructose units, wherein the inulins are coupled to a fluorescent label.

5. Kit (110) according to Claim 4, wherein the indicator substance is a mixture of inulins comprising from 3 to 8 fructose units, wherein the inulins are coupled to a fluorescent label.

6. Kit (110) according to Claim 4 or 5, wherein the at least one radiation source in the sensor plaster (116) comprises at least one light source (142) comprising an organic light-emitting material (170), more particularly an organic light-emitting diode.

7. Kit (110) according to any of Claims 4 to 6, wherein the at least one detector (146) in the sensor plaster (116) comprises at least one detector (146) comprising at least one organic semiconducting material (168, 178, 180), more particularly an organic photodetector.

8. Kit (110) according to any of Claims 4 to 7, wherein the sensor plaster (116) furthermore comprises at least one driving electronic unit (152), wherein said driving electronic unit (152) of the sensor plaster (116) comprises at least one organic component, more particularly an organic conductor track and/or an organic field effect transistor.

9. Kit (110) according to Claim 8, wherein the driving electronic unit (152) of the sensor plaster (116) is designed to control a temporally resolved measurement of the sensor plaster (116).

10. Kit (110) according to any of Claims 4 to 9, wherein the adhesive surface (138) of the sensor plaster (116) laterally encloses the detector (146), wherein, with sensor plaster (116) stuck onto the body surface, this prevents ambient light from being able to pass to the detector (146).

11. Kit (110) according to any of Claims 4 to 10, wherein the sensor plaster (116) is produced in a layer design and has at least two different layer planes.

12. Kit (110) according to any of Claims 4 to 11, furthermore comprising at least one reader (118), wherein the reader (118) is designed to interact with the sensor plaster (116), more particularly to initiate and/or read out a measurement of the organ function by means of the sensor plaster (116) .

13. Kit (110) according to Claim 12, wherein the reader (118) comprises at least one radio-frequency transmitter (126) designed to emit a radio-frequency pulse, wherein the sensor plaster (116) is designed to start, upon reception of the radio-frequency pulse, a measurement with emission of the interrogation light (162) and reception of the response light (176).

## Revendications

1. Utilisation d'une substance indicatrice marquée par fluorescence pour la préparation d'un agent diagnostique destiné à la détermination du débit de filtration glomérulaire (DFG) sur le corps d'un patient humain ou animal, la substance indicatrice étant un mélange d'inulines qui présentent 11 à 15 ou 3 à 8 unités de fructose, les inulines étant couplées à un marqueur fluorescent.

2. Utilisation selon la revendication 1, la substance indicatrice étant un mélange d'inulines qui présentent 3 à 8 unités de fructose, les inulines étant couplées à un marqueur fluorescent.

3. Utilisation selon la revendication 1 ou 2, le DFG étant déterminé pour le diagnostic d'un trouble existant de la fonction rénale, pour la détermination du risque de troubles futurs de la fonction rénale, pour la surveillance ("monitoring") lors de maladies, d'interventions thérapeutiques ou de thérapies qui peuvent provoquer des troubles de la fonction rénale ou pour la détermination de la dose individuelle pour des médicaments qui sont excrétés via les reins.

4. Kit (110) pour la mesure transcutanée d'une fonction d'un organe, en particulier d'une fonction rénale, comprenant
(a) au moins une substance indicatrice (112), la substance indicatrice (112) pouvant être introduite dans un corps, l'évolution dans le temps de la concentration en substance indicatrice (112) dans le corps, en particulier dans un tissu corporel et/ou un liquide corporel, étant utilisable comme indicateur pour la fonction de l'organe, la substance indicatrice (112) comprenant au moins un marqueur, le marqueur étant conçu pour émettre au moins une lumière de réponse (176) lors de l'irradiation d'au moins une lumière interrogatrice (162) de la source de rayonnement du pansement à capteur (116) ; et
(b) au moins un pansement à capteur (116) comprenant au moins un élément support (134) souple, présentant au moins une surface adhésive (138) pouvant être collée sur une surface corporelle, comprenant en outre au moins une source de rayonnement, en particulier une source lumineuse (142), la source de rayonnement étant conçue pour irradier la surface corporelle par au moins une lumière interrogatrice (162), la lumière interrogatrice étant une lumière utilisable pour la détection de la substance indicatrice, comprenant en outre au moins un détecteur (146), le détecteur (146) étant conçu pour détecter au moins une lumière de réponse (176) irradiée provenant de la surface corporelle,
la substance indicatrice étant un mélange d'inulines qui présentent 11 à 15 ou 3 à 8 unités de fructose, les inulines étant couplées à un marqueur fluorescent.

5. Kit (110) selon la revendication 4, la substance indicatrice étant un mélange d'inulines qui présentent 3 à 8 unités de fructose, les inulines étant couplées à un marqueur fluorescent.

6. Kit (110) selon la revendication 4 ou 5, ladite au moins une source de rayonnement dans le pansement à capteur (116) comprenant au moins une source lumineuse (142) présentant un matériau (170) électroluminescent organique, en particulier une diode électroluminescente organique.

7. Kit (110) selon l'une quelconque des revendications 4 à 6, ledit au moins un détecteur (146) dans le pansement à capteur (116) comprenant au moins un détecteur (146) présentant au moins un matériau semi-conducteur organique (168, 178, 180), en particulier un photodétecteur organique.

8. Kit (110) selon l'une quelconque des revendications 4 à 7, le pansement à capteur (116) présentant en outre au moins une électronique de commande (152), ladite électronique de commande (152) du pansement à capteur (116) comprenant au moins un élément organique, en particulier une piste conductrice organique et/ou un transistor à effet de champ organique.

9. Kit (110) selon la revendication 8, l'électronique de commande (152) du pansement à capteur (116) étant conçue pour commander une mesure à résolution temporelle du pansement à capteur (116).

10. Kit (110) selon l'une quelconque des revendications 4 à 9, la surface adhésive (138) du pansement à capteur (116) entourant le détecteur (146) latéralement, l'arrivée de lumière environnante au niveau du détecteur (146) étant empêchée sur le pansement à capteur (116) collé sur la surface corporelle.

11. Kit (110) selon l'une quelconque des revendications 4 à 10, le pansement à capteur (116) étant fabriqué selon un mode de construction par stratification et présentant au moins deux plans de couche différents.

12. Kit (110) selon l'une quelconque des revendications 4 à 11, comprenant en outre au moins un appareil de lecture (118), l'appareil de lecture (118) étant conçu pour coopérer avec le pansement à capteur (116), en particulier pour initier et/ou lire une mesure de la fonction de l'organe au moyen du pansement à capteur (116).

13. Kit (110) selon la revendication 12, l'appareil de lecture (118) comprenant au moins un transmetteur haute fréquence (126), qui est conçu pour émettre une impulsion haute fréquence, le pansement à capteur (116) étant conçu pour démarrer, lors de la réception de l'impulsion haute fréquence, une mesure par l'émission de la lumière interrogatrice (162) et la réception de la lumière de réponse (176).
